# EUROPÄISCHE PATENTSCHRIFT

(11) **EP 2 374 444 B1**
(45) Veröffentlichungstag und Bekanntmachung des Hinweises auf die Patenterteilung: **01.03.2017**
(21) Anmeldenummer: 11162128.0
(22) Anmeldetag: 12.04.2011
(51) Int. Cl.: A61K 6/083, A61K 6/00

(54) **Dentale Abdeckmasse**
Dental covering material
Masse de recouvrement dentaire

(30) Priorität: 12.04.2010 DE 102010003881
(43) Veröffentlichungstag der Anmeldung: 12.10.2011
(73) Patentinhaber: VOCO GmbH, 27472 Cuxhaven (DE)
(72) Erfinder: Maletz, Reinhard, 27472, Cuxhaven (DE); Krumme, Wigand, 27476 Cuxhaven (DE); Plaumann, Manfred Thomas, 27472 Cuxhaven (DE)
(74) Vertreter: Eisenführ Speiser

(56) Entgegenhaltungen:
- EP-B1- 1 720 506
- WO-A1-2008/096182
- DE-A1- 1 795 395
- DE-A1- 3 010 373
- DE-C1- 4 233 886
- US-A- 4 490 497
- US-B1- 6 305 936
- DE WIJN J R: "Reduction of maximum temperature in the polymerization of cold- and heat-curing acrylic resins.", JOURNAL OF BIOMEDICAL MATERIALS RESEARCH NOV 1974, vol. 8, no. 6, November 1974 (1974-11), pages 421-434, ISSN: 0021-9304

## Beschreibung

Im Zusammenhang mit der Erfindung offenbart wird primär eine photopolymerisierbare dentale Zusammensetzung zur Herstellung einer Isolierung von zu behandelnder Zahnsubstanz und eines Schutzes des umgebenden Zahnfleisches und/oder eines oder mehrerer benachbarter Zähne vor dentalen Behandlungsmitteln. Ferner offenbart wird ein Kit umfassend eine solche dentale Zusammensetzung, ein Verfahren zum Herstellen einer entsprechenden Schutzschicht, ein kosmetisches Behandlungsverfahren eines oder mehrerer Zähne sowie eine entsprechende Zusammensetzung als Abdeckmasse.
Die Erfindung wird in den beigefügten Patentansprüchen definiert.
Unter einer erfindungsgemäßen "dentalen Abdeckmasse" wird im Rahmen des vorliegenden Textes eine photopolymerisierbare dentale Zusammensetzung auf der Basis radikalisch härtbarer Acrylat-/Methacrylatsysteme zur Applikation auf und zum Isolieren und Schützen von Zahnfleisch und/oder Zähnen vor bzw. während einer Zahnbehandlung verstanden.

Es gibt eine Reihe zahnärztlicher Behandlungsverfahren, bei denen aggressive chemische Substanzen in der Mundhöhle eingesetzt werden, die gesundheitsgefährdend und schädigend für die Mundschleimhaut sein können.

So wird beispielsweise bei dem Legen einer Kunststofffüllung die sogenannte "Säure-Ätz-Technik" angewandt, bei der sowohl der Zahnschmelz als auch das Dentin einer präparierten Kavität zum Aufbau einer Adhäsion vor Auftragen eines Bondings konditioniert wird. Als Konditionierungsmittel kommen z.B. Phosphorsäure, Maleinsäure, Salpetersäure, Zitronensäure oder Oxalsäure zum Einsatz. In der Regel verwendet man heutzutage eine 35 - 40 Gew.-%ige Phosphorsäure. Um an dem präparierten Schmelz ein ausreichendes Ätzmuster erhalten zu können, lässt man die Säure zunächst 15 Sekunden am Schmelz einwirken und appliziert die Säure dann auf das Dentin, wo man sie weitere 15 Sekunden wirken lässt. Vor dem weiteren Behandlungsgang werden die Säurereste dann weggespült und abgesaugt. Das Zahnfleisch muss als Weichgewebe hierbei von den Säureresten isoliert sein.

Oft liegen die Präparationen an den Zahnrändern oder an den Zahnhälsen. In diesen Fällen befinden sich der Nachbarzahn bzw. das Weichgewebe in unmittelbarer Nähe des Behandlungsorts. Selbstverständlich müssen in solchen Fällen der Nachbarzahn bzw. das Zahnfleisch vor dem Ätzmittel geschützt werden.

Ein anderes zahnärztliches Verfahren, bei der die Mundhöhle mit aggressiven chemischen Substanzen in Berührung kommt, ist die oxidative Zahnaufhellung, auch "Bleaching" genannt, ein Verfahren, das sich mittlerweile zu einem festen Bestandteil im Bereich der ästhetischen (kosmetischen) Zahnbehandlung entwickelt hat. Die aufhellende Wirkung wird hierbei von Oxidationsmitteln wie Wasserstoffperoxid, Perboraten oder Carbamidperoxid erzielt. Zumeist werden peroxidhaltige Gele verwendet.

Zahnschmelz und Dentin sind durch im Zahn eingelagerte Farbstoffe verfärbt. Die eingesetzten Peroxide dringen in die Zahnsubstanz ein und oxidieren die eingelagerten Farbstoffe. Zurück bleiben farblose Fragmente, Schmelz und Dentin sind entfärbt. Die reaktive Spezies in diesem Prozess sind freie Radikale, die in der Lage sind, auch körpereigene Strukturen anzugreifen und zu zerstören. Bei der Aufhellung vitaler Zähne wird das Bleichmittel auf die äußeren Zahnoberflächen bis nahe an den Zahnfleischsaum appliziert. Aus frühen klassischen Untersuchungen ist bekannt, dass bei einem hohen prozentualen Teil der Patienten während der Bleichbehandlung Gingivairritationen auftraten. Um diesen Reizungen vorzubeugen, ist eine schützende Abdeckung der Mundschleimhaut unumgänglich.

Es gibt zahlreiche andere zahnärztliche Behandlungsverfahren, bei denen der behandelte Zahn oder gleich mehrere behandelte Zähne in einem trockenen Zustand gehalten werden müssen. Hierzu muss dieser Behandlungsort flüssigkeitsdicht vom umgebenden Hart- und Weichgewebe abgeschirmt bleiben.

Die klassische Methode zur Abschirmung und Isolierung von zu behandelnder Zahnsubstanz vom restlichen Mundraum sowie zum Schutz von leicht verletzlichem Weichgewebe ist die sogenannte "Kofferdam-Technologie", die unter Verwendung eines Spanntuchs aus Gummi, das als Kofferdam bezeichnet wird, entwickelt wurde. Hierbei perforiert der Zahnarzt die Kofferdammembran an den passenden Stellen mit Hilfe einer Lochzange und stülpt dann die perforierte Membran über den zu behandelnden Zahn. Der Kofferdam wird dann mittels eines Kofferdam Rahmens gespannt, so dass der Zahn vom umgebenden Mundraum abgeschirmt ist. Die Nachteile der "Kofferdam-Technologie" sind vielfältig. So verspürt der Patient in der Regel ein unangenehmes Gefühl durch die Druckspannung der Metallklammern am Zahn. Er kann den Mund nicht mehr schließen und muss durch die Nase atmen. Das Verfahren ist äußerst aufwendig und zeitintensiv für den Zahnarzt sowie sehr empfindlich gegenüber Anwendungsfehler. Ist beispielsweise das Loch nicht in der richtigen Größe im Spanntuch ausgestanzt, so wird der Kofferdam nicht genau und stramm genug entlang der Zahnfleischgrenze sitzen und für eine ausreichende Abdichtung sorgen. Es besteht ebenfalls die Gefahr, dass das Tuch beim Spannen oder während der Behandlung reißt, so dass die abschirmende Wirkung verloren geht und beispielsweise aggressive chemische Substanzen ungehindert zu den Weichgewebeteilen des Mundes gelangen können und dort Schädigungen verursachen.

Bekanntlich entsteht bei der radikalischen Polymerisation lichthärtbarer Methacrylatsysteme eine große Wärmemenge, die abgeführt werden muss, insbesondere dann, wenn solche Zusammensetzungen beispielsweise auf das Weichgewebe des Mundinnenraums eingesetzt werden sollen. Es sollte sichergestellt sein, dass es im Mundraum des Patienten durch die freigesetzte Wärmemenge nicht zu Schmerzen oder gar Verbrennungen kommen kann.

In der US 6,305,936 B1 werden Zusammensetzungen auf Basis radikalisch härtbarer Methacrylatsysteme und Verfahren vorgeschlagen, die die oben genannten Nachteile der Kofferdamtechnik überwinden sollen. Zur Reduzierung der Wärmemenge werden in der US 6,305,936 B1 nicht reaktive Weichmacher wie Polyole oder Mineralöl verwendet. Nachteilig dabei ist beispielsweise, dass der Einsatz nicht reaktiver Weichmacher den Ablauf einer homogenen Vernetzung stört und es so zum Vorliegen nicht umgesetzter Monomere kommen kann. Monomere Methacrylate weisen zytotoxische Eigenschaften auf und können sensibilisierend auf das Gewebe wirken.

US 6,800,671 B1 betrifft Isolationsmaterial von zu behandelnder Zahnsubstanz und des umgebenden Zahnfleisches. Die dort beschriebenen Zusammensetzungen enthalten ungesättigte, härtbare Verbindungen, die einen Index an Ungesättigtheit von wenigstens 500 aufweisen. Bevorzugte härtbare Verbindungen sind gemäß US 6,800,671 B1 Dimethacrylatpolyetherurethanoligomere mit Molekulargewichten zwischen 1.000 und 3.000 g/mol. Zusätzlich zu diesen multifunktionell ungesättigten Verbindungen enthalten die dort beschriebenen Zusammensetzungen ein Härtungsmittel sowie eine haftfördernde Verbindung. Um eine verarbeitbare Konsistenz der Zusammensetzung gewährleisten zu können, kann der Zusammensetzung aus US 6,800,671 B1 ein nichthärtbares Verdünnungsmittel wie beispielsweise Mono-, Di- oder Triglyceride oder Pflanzenöl beigegeben werden.

WO 2008/096182 betrifft ein photopolymerisierbares Material zur Isolierung und zum Schutz des Zahnfleisches, beispielsweise während des Zahnbleichens. Die dort beschriebene Zusammensetzung umfasst wenigstens ein Monomer, wenigstens ein Polymerisationsmittel, wenigstens ein inertes Rohmaterial und als weiteren Bestandteil wenigstens eine Verbindung mit antioxidativen, antientzündlichen oder sedativen Eigenschaften. In einigen Beispielen wird Tocopherylacetat, eine Verbindung, die sich nicht an einer radikalischen Polymerisation beteiligt, in einer Menge bis zu 25 Gew.-% eingesetzt. Auch bei den Zusammensetzungen gemäß WO 2008/096182 ist zu erwarten, dass diese keine homogenen Polymerisate bilden und eine zu hohe Menge an nicht umgesetztem Monomer im ausgehärteten Material vorhanden sein wird.

In der US 5,900,245 wird ebenfalls ein System zur Herstellung einer Abschirmung von Weichgewebe angegeben, wobei die Gewebeoberfläche mit einer Kunststoffzusammensetzung beschichtet wird. Dort wird zunächst ein Polymerisationsinitiator auf die Gewebeoberfläche aufgebracht und anschließend eine härtbare Zusammensetzung auf die bereits präparierte Gewebeoberfläche appliziert und lichtgehärtet.

EP 1 553 915 B1 offenbart die Verwendung einer elastomeren, am Zahnfleisch anhaftenden Abdeckmasse, die als zweikomponentiges System, gemischt bei Umgebungstemperatur, auf dem zu schützenden Gewebe selbsthärtend vernetzt. Dieses System bedient sich der Silikonchemie.

DE 42 33 886 C1 offenbart polymerisierbare Konditionierungsmittel auf Methacryl-Basis und ein Verfahren zur Vorbehandlung der Oberfläche von Formkorpern aus Polyacrylat-, Polymethacrylat- und Polycarbonat-Kunststoffen vor dem Auftragen von polymerisierbarem Methacrylat-Material und die Verwendung des Konditionierungsmittels. Das Konditionierungsmittel kann monocyclischen Terpenkohlenwasserstoff enthalten. Eine Verwendung des offenbarten Konditionierungsmittels zur Herstellung einer Isolierung von zu behandelnder Zahnsubstanz und eines Schutzes des umgebenden Zahnfleisches und/oder eines oder mehrerer benachbarter Zähne vor dentalen Behandlungsmitteln ist nicht beschrieben; ein entsprechender Einsatz erscheint aufgrund der Verwendung von als toxisch einzustufenden Alkylmethacrylaten auch ausgeschlossen.DE 30 10 373 A1 offenbart Verfahren zur Polymerisation von Methacrylsauremethylester oder dessen Gemischen mit weiteren Vinylmonomeren in Gegenwart von Enolethern. Die nach dem offenbarten Verfahren hergestellten Polymerisate werden zwar als besonders geeignet für den Einsatz im Dentalbereich bezeichnet, beispielsweise zur Herstellung von Prothesen nach dem Pulver-Flüssigkeitsverfahren. Nicht offenbart werden allerdings Zusammensetzungen, die zur Herstellung einer (ausreichend elastischen und nicht toxischen) Isolierung von zu behandelnder Zahnsubstanz und eines Schutzes des umgebenden Zahnfleisches und/oder eines oder mehrerer benachbarter Zähne vor dentalen Behandlungsmitteln geeignet erscheinen.

DE 27 27 480 A1 offenbart schlagzähe, glasartige Kunststofflegierungen aus Polymethacrylaten und aliphatischen Polyurethanharnstoffen, Es werden Verfahren zu deren Herstellung offenbart, in denen Monomerenmischungen eingesetzt werden, die schwefelhaltige Molekulargewichtsregler umfassen. Nicht offenbart werden Zusammensetzungen für den dentalen Bereich und insbesondere auch nicht Zusammensetzungen, die zur Herstellung einer (ausreichend elastischen und nicht toxischen) Isolierung von zu behandelnder Zahnsubstanz und eines Schutzes des umgebenden Zahnfleisches und/oder eines oder mehrerer benachbarter Zähne vor dentalen Behandlungsmitteln geeignet erscheinen.

DE 17 95 395 A offenbart Verfahren zur Herstellung von Polymerisaten und Mischpolymerisaten von (Meth-}Acrylsaureestern und von Acrylnitril in Gegenwart von Polymerisationskatalysatoren und von Verbindungen mit einem Sechserring, der zwei nichtkonjugierte Doppelbindungen aufweist, von denen eine semicyclisch (exocyclisch) sein kann. Die speziell offenbarten Polymerisationskatalysatoren sind thermische Katalysatoren, Photoinitatoren oder photopolymerisierbare dentale Zusammensetzungen sind nicht offenbart.

US 4,490,497 A offenbart Zusammensetzungen für chirurgischen Zement, die bei der Herstellung dentaler Prothesen eingesetzt werden können. Eine Flüssigkomponente der Zusammensetzung umfasst als "chain stopper" beispielsweise ein doppelt ungesättigtes monocyclisches Terpen oder ein einfach ungesättigtes bicyclisches Terpen. Nicht offenbart sind Zusammensetzungen, die zur Herstellung einer (ausreichend elastischen und nicht toxischen) Isolierung von zu behandelnder Zahnsubstanz und eines Schutzes des umgebenden Zahnfleisches und/oder eines oder mehrerer benachbarter Zähne vor dentalen Behandlungsmitteln geeignet erscheinen.

EP 1 720 506 B1 offenbart ein gefülltes und polymerisierbares Dentalmaterial sowie ein Verfahren zu dessen Herstellung. Als Anwendungsbeispiele werden genannt: Zahnfüllungsmaterialien, Stumpfaufbaumaterialien, Materialien für provisorische Kronen und Brücken, Zahnzemente Adhäsive, Materialien für künstliche Zähne, Verblendmaterialien, Versiegelungsmaterialien und Dentallacke. Es wird offenbart, dass das Dentalmaterial zur Einstellung bestimmter Eigenschaften Additive bzw. Modifikatoren enthalten kann; in einer langen Liste von Beispielen findet sich auch "Terpinene". Nicht offenbart werden allerdings Zusammensetzungen, die zur Herstellung einer (ausreichend elastischen und nicht toxischen) Isolierung von zu behandelnder Zahnsubstanz und eines Schutzes des umgebenden Zahnfleisches und/oder eines oder mehrerer benachbarter Zähne vor dentalen Behandlungsmitteln geeignet erscheinen.

Bei den Systemen der radikalischen Polymerisation lichthärtbarer Massen wurde das Problem der hohen Wärmeentwicklung in allen Fällen dadurch angegangen, entweder der Zusammensetzung nicht-reaktive Zusätze beizugeben und/oder Monomere hohen Molekulargewichts, d.h. Verbindungen mit einem geringen Doppelbindungsanteil, zu verwenden. Der Zusatz nicht an der Vernetzung beteiligter Substanzen stört zum einen den homogenen Verlauf einer radikalischen Polymerisation und führt überdies bekanntlich dazu, dass die nicht im Netzwerk eingebundenen Verbindungen aus der organischen Matrix auswandern und das Weichgewebe schädigen können. Werden indes Monomere hohen Molekulargewichts verwendet, so wird die Mobilität der funktionellen Gruppen reduziert und die Wahrscheinlichkeit vermindert, dass jede reaktive Gruppe auch einen entsprechenden Reaktionspartner findet. Die mechanischen Eigenschaften des Schutzfilms werden herabgesetzt und die Qualität des Isolationsmaterials wird leiden.

Bei den chemisch härtenden Systemen auf Silikonbasis gibt es die entscheidenden Vorteile, dass sie biokompatibel sind und keine Wärmeentwicklung während der Aushärtung zeigen. Allerdings ist die Akzeptanz der Silikone bei den Zahnärzten nicht sehr ausgeprägt, da die Zahnärzte in erster Linie an schnellen Aushärtevorgängen interessiert sind, die ihnen die Verwendung lichthärtbarer Massen liefert.

Aufgabe der vorliegenden Erfindung ist es daher, eine Zusammensetzung zur Herstellung einer Isolierung von zu behandelnder Zahnsubstanz und eines Schutzes des umgebenden Zahnfleisches und/oder eines oder mehrerer benachbarter Zähne vor dentalen Behandlungsmitteln auf der Basis lichthärtbarer Methacrylat-/Acrylatsysteme zur Verfügung zu stellen, die nicht auf die Zugabe nicht-reaktiver Additive oder die ausschließliche Verwendung von Monomeren hohen Molekulargewichts angewiesen ist. Ein solches Material sollte schnell aushärten, keine nicht an der Polymerisation beteiligten Verbindungen an das umgebende Gewebe abgeben, hohe Vernetzungsraten aufweisen und somit nach Aushärtung des Materials keine nachteiligen Mengen nicht umgesetzter Monomere mehr enthalten, die später einen schädlichen Einfluss auf das Gewebe nehmen können, ein regelmäßig aufgebautes Netzwerk liefern und somit gute mechanische Eigenschaften zeigen, an dem Gewebe haften und dennoch leicht wieder entfernbar sein.

Die Aufgabe wird durch eine Zusammensetzung gemäß Anspruch 1 gelöst. Vorteilhafte Ausgestaltungen der Erfindung sind in den Unteransprüchen angegeben.

Die vorliegende Erfindung betrifft somit eine photopolymerisierbare dentale Zusammensetzung als dentale Abdeckmasse zur Applikation auf und zum Isolieren und Schützen von Zahnfleisch und/oder Zähnen während einer Zahnbehandlung, umfassend:
(a) ein oder mehrere photopolymerisierbare Monomere ausgewählt aus der Gruppe bestehend aus Acrylaten und Methacrylaten, vorzugsweise der Gruppe der Methacrylate,
(b) ein oder mehrere Photoinitiatoren, vorzugsweise ausgewählt aus der Gruppe bestehend aus alpha-Diketonen, Benzoinalkylethern, Thioxanthonen, Benzophenonen, Acylphosphinoxiden, Acetophenonen, Ketalen, Titanocenen, Boraten und sensibilisierenden Farbstoffen,
(c) ein oder mehrere Molekulargewichtsregler ausgewählt aus der Gruppe bestehend aus α-Terpinen und γ-Terpinen (dies sind Verbindungen, die mit einem Radikal eines Monomers des Bestandteils (a) unter Abstraktion eines H-Radikals aus dem Molekulargewichtsregler in Allylposition umsetzbar sind),
sowie gegebenenfalls ein oder mehrere weitere Additive.

Überraschenderweise wurde gefunden, dass es gelingt, lichthärtbare Massen auf der Basis photopolymerisierbarer Monomere aus der Gruppe der Acrylate und Methacrylate in Form von Abdeckmassen zur Herstellung einer Isolierung von zu behandelnder Zahnsubstanz und eines Schutzes des umgebenden Zahnfleisches und/oder eines oder mehrerer benachbarter Zähne vor dentalen Behandlungsmitteln (insbesondere dentalen Bleichmitteln) zur Verfügung zu stellen, wenn man den Massen die erfindungsgemäß einzusetzenden Molekulargewichtsregler beigibt. Die erfindungsgemäßen Zusammensetzungen härten schnell aus, geben keine großen Wärmemengen an die Umgebung ab, weisen hohe Vernetzungsraten auf, enthalten vorzugsweise keine nicht-reaktiven Bestandteile und enthalten vorzugsweise nicht ausschließlich Monomere hohen Molekulargewichts.

Die erfindungsgemäßen Zusammensetzungen enthalten vorzugsweise ein oder mehrere niedrigmolekulare photopolymerisierbare Monomere, bevorzugt ein oder mehrere photopolymerisierbare Monomere mit einem Molekulargewicht von < 700 g/mol, besonders bevorzugt mit einem Molekulargewicht von < 600 g/mol.

In einer weiter bevorzugten Ausführungsform enthält eine erfindungsgemäße Zusammensetzung eine Gesamtmenge an niedrigmolekularen photopolymerisierbaren Monomeren von mehr als 50 Gew.-%, bezogen auf den Gesamtanteil an Monomeren einer erfindungsgemäßen Zusammensetzung.

Es war überraschend, als festgestellt wurde, dass die erfindungsgemäß einzusetzenden Molekulargewichtsregler (siehe dazu unten) eine hochwirksame regelnde Wirkung in einer erfindungsgemäßen dentalen Abdeckmasse entwickeln. Der beobachtete Temperaturverlauf einer erfindungsgemäßen Abdeckmasse ist gegenüber der Temperaturentwicklung einer Formulierung ohne Molekulargewichtsregler in seinem Maximum um mehrere Grad Celsius reduziert. Die abgegebene Wärmemenge wird bei Anwesenheit eines geeigneten Molekulargewichtsreglers nicht ungebremst und unkontrolliert "auf einen Schlag" abgegeben, sondern "geregelt", d.h. kontrolliert, in einer Weise, dass hohe thermische Belastungen oder gar Verbrennungen auf dem Gewebe im Mundraum ausgeschlossen werden können. Gleichzeitig muss der Regler das Molekulargewicht des Polymerisats in einer Weise herabsetzen, dass das Material gut am Gewebe anhaftet und dennoch leicht wieder entfernbar ist.

Gegenüber dem Stand der Technik wird hier somit eine Abdeckmasse zur Herstellung einer Isolierung von zu behandelnder Zahnsubstanz und eines Schutzes des umgebenden Zahnfleisches und/oder benachbarter Zähne vor dentalen Behandlungsmitteln angegeben, die vorzugsweise zwei, drei oder sämtliche der folgenden Eigenschaften aufweist:
1.) die Abdeckmasse ist im Wesentlichen frei, vorzugsweise frei, von nicht-reaktiven organischen Verbindungen, die an die Umgebung abgegeben werden können und die einem homogenen Aufbau des Polymerisats im Wege stehen,
2.) die Abdeckmasse härtet schnell und homogen aus und erzielt hohe Umsatzraten, wobei die Molekulargewichte der eingesetzten Monomere in einer Größenordnung liegen, die eine ausreichende Mobilität der reaktiven Spezies zulassen, so dass nicht zu erwarten ist, dass in der ausgehärteten Masse nachteilige Mengen noch nicht umgesetzter Monomere vorliegen, die später einen schädlichen Einfluss auf das Gewebe nehmen können,
3.) die ausgehärtete Isolierung weist ein homogenes und regelmäßig aufgebautes Netzwerk auf, das im Vergleich zu einer Abdeckmasse ohne Regler ein geringeres Molekulargewicht aufweist, so dass die ausgehärtete Masse weichere Eigenschaften besitzt, die dazu führen, dass sie bei einem guten mechanischen Profil flexibel am Gewebe anhaftet und dennoch leicht wieder entfernt werden kann,
4.) die Abdeckmasse löst das große Problem der auf der Methacrylat-/Acrylatchemie basierenden Systeme dentaler Abdeckmassen, nämlich die Freisetzung großer Wärmemengen, ohne andere Nachteile dafür in Kauf nehmen zu müssen.

Molekulargewichtsregler (im Folgenden auch kurz als Regler bezeichnet) sind an sich bekannt und kommerziell erhältlich. Man benutzt sie beispielsweise bei der Lösungspolymerisation von Olefinen, bei der Emulsionspolymerisation von Methacrylaten oder zur Herstellung von Formkörpern aus PMMA-Formmassen (PMMA = Polymethylmethacrylat) durch Formpressen oder Spritzgießen.

Molekulargewichtsregler stellen sogenannte Übertragungsreagentien dar, die in einer freien radikalischen Reaktion Transferreaktionen eingehen und mechanistisch H-Abstraktion und Übertragung der Radikalfunktion auf den Regler beinhaltet. Der Regler findet sich dann aufgrund seiner Funktion in Form von Endgruppen im vernetzten Polymerisat wieder.

Übliche Regler sind beispielsweise Aldehyde und Ketone, wie Formaldehyd, Acetaldehyd, Propionaldehyd, *n*-Butyraldehyd, Isobutyraldehyd, Methylethylketon, Aceton, Methylisobutylketon, Ameisensäure, Ammoniumformiat, Hydroxylammoniumsulfat und Hydroxylammoniumphosphat, Verbindungen, die Schwefel in organisch gebundener Form enthalten, wie Di-*n*-butylsulfid, Di-*n*-octylsulfid, Diphenylsulfid, Diisopropyldisulfid, Di-*n*-butyldisulfid, Di-*n*-hexyldisulfid, Diacetyldisulfid und Di-*tert.-*butyltrisulfid, Verbindungen, die Schwefel in Form von SH-Gruppen aufweisen, wie *n*-Butylmercaptan, *n*-Hexylmercaptan und *n*-Dodecylmercaptan, Octadecylmercaptan, weitere Schwefelverbindungen wie Hydrogensulfite, Disulfite, Verbindungen wie Mercaptoethanol, Mercaptobutanol, Mercaptoessigsäure, 3-Mercaptopropionsäure, Mercaptobernsteinsäure, Thioglycerin, Thioglykolsäure, Diethanolsulfid, Thiodiglykol, Ethylthioethanol, 2,2,4,6,6-Pentamethylheptan-4-thiol, 2,2,4,6,6,8,8-Heptamethylnonan-4-thiol, Thioharnstoff, Dimethylsulfoxid, Ethylhexylthioglycolat, Pentaerythrittetrathioglycolat, Mercaptopropyltrimethoxysilan, dann Allylverbindungen wie Allylalkohol, Allylbromid, oder Benzylverbindungen wie Benzylchlorid oder Alkylhalogenide wie Chloroform, Bromtrichlormethan oder Tetrachlormethan, Tetrabrommethan, Methylenchlorid, weiterhin niedere und höhermolekulare einwertige oder mehrwertige Alkohole wie Methanol, Ethanol, *n*-Propanol, Isopropanol, *tert*.-Butanol, sec-Butanol, *n*-Butanol, Amylalkohol, Cyclohexanol, Octanol, Dodecanol, 1-Ethylhexanol, Glycerin, Stearylalkohol, Oleylalkohol, Hydroxyethylmethacrylat oder Amine wie Triethylamin sowie Toluol oder Ethylbenzol.

Die oben genannten Regler sind für den hier vorgesehenen Einsatz jedoch ungeeignet. So weisen schwefelhaltige Molekulargewichtsregler einen intensiven und typisch unangenehmen Geruch auf, sie scheiden als Bestandteil eines dentalen Materials aus. Ferner wurde gefunden, dass Alkohole, nieder- oder höhermolekular, ein- oder mehrwertig, in den Zusammensetzungen keine regelnde Wirkung entfalten und unwirksam bleiben. Gleiches gilt für Aldehyde und Ketone sowie für Säuren.

Der genaue Grund für den erfindungsgemäßen Erfolg bei Verwendung der oben angegebenen Molekulargewichtsregler (c) ist nicht im Detail bekannt. Vermutlich besteht jedoch eine besondere, vorab nicht zu erwartende Wechselwirkung zwischen dem oder den Monomeren und dem oder den Molekulargewichtsreglern, die es ermöglicht, dass bei der Photopolymerisation der erfindungsgemäßen Abdeckmasse der Molekulargewichtsregler (c) in einer Art und Weise und in einem Maße Einfluss auf den Polymerisationsverlauf nimmt, dass Kettenlänge, Vernetzung und Restmonomergehalt des sich bildenden Polymers derart gestaltet sind, dass die gewünschten Eigenschaften erhalten werden.

Grundsätzlich geeignete Molekulargewichtsregler sind beispielsweise diverse Terpene, insbesondere Terpinene (*α*-Terpinen, *β*-Terpinen, *γ*-Terpinen), Phellandrene (*α-*Phellandren, *β*-Phellandren) und Terpinolen (auch *δ*-Terpinen genannt), 1,4-Cyclohexadien (gegebenenfalls substituiert), 1,3-Cyclohexadien (gegebenenfalls substituiert), 1,4-Dihydronaphtalin, 1,4,5,8-Tetrahydronaphthalin, 2,5-Dihydrofuran oder dimeres *α*-Styrol (2,4-Diphenyl-4-methyl-1-penten) sowie Linolsäure und α-Linolensäure.

Erfindungsgemäß als Bestandteil (c) einer erfindungsgemäßen Abdeckmasse einzusetzende Molekulargewichtsregler sind weiter unten definierte. Diene, die durch Abstraktion eines Allyl-H-Atoms ein delokalisiertes Elektronensystem ausbilden können, das sich über 5 C-Atome erstreckt.

Erfindungsgemäß eingesetzte Regler sind α-Terpinen und γ-Terpinen, insbesondere bevorzugt ist γ-Terpinen.

In einer erfindungsgemäßen Abdeckmasse können selbstverständlich auch Mischungen von Molekulargewichtsreglern eingesetzt werden.

Die Zugabe der erfindungsgemäß einzusetzenden Molekulargewichtsregler, des Bestandteils (c) zu dentalen Abdeckmassen ist bisher unbekannt.

In EP 1 720 506, WO 2009/083168 A1, DE 10 2008 028 306 A1, EP 1 872 767 A1 sowie EP 2 070 506, die sämtlich keine Abdeckmassen im Sinne der vorliegenden Erfindung betreffen, werden in der jeweils angegebenen Liste der Stabilisatoren unter anderem Terpinene aufgeführt.

Erfindungsgemäße dentale Zusammensetzungen umfassen vorzugsweise keinen schwefelhaltigen Molekulargewichtsregler.

Erfindungsgemäß bevorzugte dentale Zusammensetzungen umfassen
Bestandteil (a) in einer Menge von 50 bis 90 Gew.-%, vorzugsweise in einer Menge von 60 bis 80 Gew.-%,
Bestandteil (b) in einer Menge von 0,01 bis 1 Gew.-%, vorzugsweise von 0,05 bis 0,5 Gew.-%, bevorzugt von 0,1 bis 0,3 Gew.-%,
   und
Bestandteil (c) in einer Menge von 0,075 bis 7,5 Gew.-%, vorzugsweise von 0,125 bis 3 Gew.-%, bevorzugt von 0,2 bis 1,5 Gew.-%,
jeweils bezogen auf die Gesamtmasse der dentalen Zusammensetzung.

### Bestandteil (a) - photopolymerisierbare Monomere

Die radikalisch lichtpolymerisierbaren Monomere können mindestens zwei ethylenische Gruppen aufweisende Substanzen sein wie beispielsweise, ohne darauf beschränkt zu sein, die in der Dentalchemie üblicherweise in Kompositmaterialien eingesetzten (Meth)acrylatmonomere.

In der Patentliteratur ist eine Vielzahl weiterer Verbindungen genannt (beispielsweise auch in der DE 39 41 629 A1, welche im Wege der Verweisung Bestandteil der vorliegenden Anmeldung ist), die allesamt Diester der Acryl- oder Methacrylsäure sind und sich zum Einsatz in einer erfindungsgemäßen Zusammensetzung eignen.

In einer bevorzugten erfindungsgemäßen dentalen Zusammensetzung umfasst Bestandteil (a)
(a) (i) ein oder mehrere Dimethacrylat-Monomere
   und/oder
(a) (ii) ein oder mehrere säuregruppenhaltige Monomere,
wobei Bestandteil (a) vorzugsweise sowohl Bestandteil (a) (i) als auch (a) (ii) umfasst.

In einer bevorzugten erfindungsgemäßen dentalen Abdeckmasse enthält Bestandteil (a) (i) ein oder mehrere Dimethacrylat-Monomere gewählt aus der Gruppe bestehend aus Ethylenglykoldimethacrylat (EGDMA), 1,6-Hexandioldimethacrylat (HEDMA), Triethylenglykoldimethacrylat (TEGDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA) und Dimethacrylaten des Dihydroxymethyltricyclo[5.2.1.0^{2,6}]decans.

Explizit bevorzugt sind auch die entsprechenden Dimethacrylate bzw. Diacrylate des Dihydroxymethyltricyclo[5.2.1.0^{2,6}]decans, wie in den Druckschriften DE 1816823, DE 2419887, DE 2406557, DE 2931926, DE 3522005, DE 3522006, DE 3703120, DE 102005021332, DE 102005053775, DE 102006060983, DE 69935794 und DE 102007034457 beschrieben, welche im Wege der Verweisung Bestandteil der vorliegenden Anmeldung sind.

Eine erfindungsgemäße dentale Abdeckmasse enthält vorzugsweise eine oder mehrere säuregruppenhaltige lichthärtbare Substanzen. Es wurde gefunden, dass es zu keiner Reizung oder sonstigen Schädigung der Gingiva kommt, wenn geringe Mengen einer säuregruppentragenden lichthärtbaren Verbindung zum Einsatz kommen. Ferner wurde gefunden, dass die Anhaftung einer Abdeckmasse am Zahn sowie ein dichter Abschluss an der Zahnhartsubstanz durch den Einsatz eines oder mehrere säuregruppenhaltigen Monomere in einer erfindungsgemäßen dentalen Zusammensetzung begünstigt wird.

In einer bevorzugten erfindungsgemäßen dentalen Zusammensetzung enthält Bestandteil (a) (ii) ein oder mehrere säuregruppenhaltige Monomere, welche eine Carbonsäure-, eine Phosphorsäure-, eine Phosphonsäure-, eine Sulfonsäure- und/oder eine Thiophosphorsäurefunktion aufweisen, vorzugsweise eine Phosphorsäurefunktion.

Die säuregruppenhaltige, lichthärtbare Verbindung kann ein polymerisierbares Monomer sein, das eine oder eine Vielzahl von Säurefunktionen in einem Molekül enthält.

Besonders bevorzugte erfindungsgemäße dentalen Zusammensetzungen sind dadurch gekennzeichnet, dass Bestandteil (a) (ii)
- ein oder mehrere eine Phosphorsäuregruppe enthaltende Monomere enthält, welche ausgewählt sind aus der Gruppe bestehend aus 2-(Meth)acryloyloxyethyldihydrogenphosphat, Bis[2-(meth)acryloyloxyethyl]hydrogenphosphat, 2-(Meth)acryloyloxyethylphenylhydrogenphosphat, 6-(Meth)-acryloyloxyhexyldihydrogenphosphat, 10-(Meth)acryloyloxydecyldihydrogenphosphat (MDP), 6-(Meth)-acryloyloxyhexylphenylhydrogenphosphat, 10-(Meth)acryloyloxydecyldihydrogenphosphat, 1,3-Di-(meth)acryloyloxypropan-2-dihydrogenphosphat, 1,3-Di-(meth)acryloyloxypropan-2-phenylhydrogenphosphat und Bis[5-(2-(meth)acryloyloxyethoxycarbonyl)heptyl]hydrogenphosphat,
   und/oder
- ein oder mehrere eine Carbonsäuregruppe enthaltende Monomere enthält, welche ausgewählt sind aus der Gruppe bestehend aus 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäureanhydrid, 11-(Meth)acryloyloxy-1,1-undecandicarbonsäure, 1,4-Di(meth)acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

Von den eine Phosphorsäuregruppe enthaltende, lichtpolymerisierbaren Monomeren ist 10-(Meth)acryloyloxydecyldihydrogenphosphat (MDP) besonders bevorzugt, da mit dieser Substanz besonders gute Ergebnisse, insbesondere sehr gute Haftwerte im Sinne der vorliegenden Erfindung, erzielt wurden.

Die Menge an säuregruppehaltigem Monomer ist vorzugsweise so ausgewählt, dass
a) ein dichter Abschluss des Zahnhalssaumes zur Gingiva erreicht wird,
b) eine moderate Haftwirkung erzielt wird, so dass die Abdeckmasse nicht bei sehr leichter (unbeabsichtigter) mechanischer Beanspruchung während der Behandlung verrutscht, und
c) eine bequeme Abziehbarkeit des Materials nach Ende der Behandlung erreicht wird.

Vorzugsweise weist eine erfindungsgemäße dentale Zusammensetzung eine moderate Haftwirkung auf, bevorzugt eine Haftkraft von 2 - 4 MPa, gemessen an Rinderschmelz nach der in ISO CD 29022 beschriebenen Methode.

Weitere geeignete säuregruppentragende Monomere sind beispielsweise in der EP 0 980 682 B1 oder der EP 0 948 955 genannt, welche im Wege der Verweisung Bestandteil der vorliegenden Anmeldung sind.

Ferner können auch die Phosphorsäureester mit Glycerindimethacrylat oder mit Hydroxyethylmethacrylat oder mit Hydroxypropylmethacrylat verwendet werden.

Sofern ein säuregruppenhaltiges Monomer des Bestandteils (a) (ii) gleichzeitig ein Dimethacrylat-Monomer des Bestandteils (a) (i) ist, wird es für die Zwecke des vorliegenden Textes als säuregruppenhaltiges Monomer des Bestandteils (a) (ii) aufgefasst, insbesondere bei quantitativen Betrachtungen der Menge an Monomer.

Ebenfalls bevorzugte erfindungsgemäße dentalen Zusammensetzungen sind dadurch gekennzeichnet, dass diese
Bestandteil (a) (i) in einer Menge von 42 bis 89,5 Gew.-%, vorzugsweise in einer Menge von 56 bis 79 Gew.-% enthält,
   und/oder
Bestandteil (a) (ii) in einer Menge von 0,5 bis 8 Gew.-%, vorzugsweise in einer Menge von 1 bis 4 Gew.-% enthält,
jeweils bezogen auf die Gesamtmasse der dentalen Zusammensetzung.

Die radikalisch lichtpolymerisierbaren Monomere können auch mindestens eine ethylenische Doppelbindung aufweisende Hydroxylverbindungen sein. Dabei können alle in der Dentalchemie üblicherweise eingesetzten Hydroxylverbindungen von Acrylaten oder Methacrylaten eingesetzt werden. Bevorzugt sind Hydroxylverbindungen von Methacrylaten, dabei wiederum bevorzugt 2-Hydroxyethylmethacrylat, 2-Hydroxypropylmethacrylat, 3-Hydroxypropylmethacrylat, 1,2-Dihydroxypropylmethacrylat, 1,3-Dihydroxypropylmethacrylat, 2,3-Dihydroxypropylmethacrylat, 2-Hydroxypropyl-1,3-dimethacrylat, 3-Hydroxypropyl-1,2-dimethacrylat, Pentaerythritoldimethacrylat, Glycerindimethacrylat, 2,2-bis[4-[3-methacryloyloxy-2-hydroxypropoxy]phenyl]propan.

Weiterhin können auch lichthärtbare Monomere mit ethylenischen Doppelbindungen auf Basis von Polysiloxanen, wie sie beispielsweise in der DE 199 03 177 oder in der DE 44 16 857 beschrieben sind, verwendet werden, welche im Wege der Verweisung Bestandteil der vorliegenden Anmeldung sind.

Die genannten lichtpolymerisierbaren Monomere können einzeln oder in Gemischen eingesetzt werden.

### Bestandteil (b) - Photoinitiatoren

Beispiele für einen Lichthärtungsinitiator schließen Katalysatoren ein, die nur photosensibilisierend wirken sowie Kombinationen aus Sensibilisator und Beschleuniger.

Beispiele für Photosensibilisatoren sind alpha-Diketone, Benzoinalkylether, Thioxanthone, Benzophenone, Acylphosphinoxide, Acetophenone, Ketale, Titanocene, sensibilisierende Farbstoffe, etc. Die Sensibilisatoren können alleine oder in Kombination angewendet werden. Konkrete Substanzbeispiele der unterschiedlichen Klassen finden sich beispielsweise in der DE 10 2006 019 092 A1 oder in der DE 39 41 629 C2, welche im Wege der Verweisung Bestandteil der vorliegenden Anmeldung sind.

Beispiele für Beschleuniger, die zusammen mit den Sensibilisatoren eingesetzt werden, sind tertiäre Amine, sekundäre Amine, Barbitursäuren, Zinnverbindungen, Aldehyde und Schwefelverbindungen. Konkrete Substanzbeispiele der unterschiedlichen Klassen finden sich in der DE 10 2006 019 092 oder in der DE 39 41 629 C2, welche im Wege der Verweisung Bestandteil der vorliegenden Anmeldung sind.

Weitere geeignete Initiatoren sowie Initiatorkombinationen sind in der DE 601 16 142 beschrieben, welche im Wege der Verweisung Bestandteil der vorliegenden Anmeldung sind.

Die im Rahmen der vorliegenden Erfindung verwendbaren Photoinitiatoren sind dadurch charakterisiert, dass sie durch Absorption von Licht im Wellenlängenbereich von 300 nm bis 700 nm, bevorzugt von 350 nm bis 600 nm und besonders bevorzugt von 380 nm bis 500 nm, gegebenenfalls in Kombination mit einem oder mehreren Coinitiatoren, die Aushärtung einer erfindungsgemäßen Zusammensetzung bewirken können.

Das Absorptionsmaximum von Campherchinon (CC) liegt bei ca. 470 nm und somit im Bereich des blauen Lichts. Campherchinon (CC) zählt zu den PI₂-Initiatoren und wird regelmäßig zusammen mit einem Coinitiator eingesetzt.

Vorzugsweise enthält eine erfindungsgemäße Abdeckmasse die Kombination eines alpha-Diketons mit eines aromatischen tertiären Amins, bevorzugt ist die Kombination von Campherchinon (CC) und Ethyl-*p*-*N,N*-dimethylaminobenzoat (DABE).

Ebenfalls bevorzugt ist die weitere Kombination des Systems "alpha-Diketon/aromatisches tertiäres Amin" mit einem Phosphinoxid, insbesondere mit dem Phenyl-bis(2,4,6-trimethylbenzoyl)phosphinoxid und/oder dem 2,4,6-Trimethylbenzoyl-diphenylphosphinoxid. Bezüglich der Strukturen geeigneter Phosphinoxide zum Einsatz in einer erfindungsgemäßen Zusammensetzung wird auf die Druckschriften DE 38 01 511 C2, DE 10 2006 050 153 A1, EP 0 184 095 B1, DE 42 31 579 C2, EP 0 366 977 B1, US 7,081,485 B2, DE 32 36 026 A1, US 2007/0027229 A1, EP 0 262 629 B1, EP 0 073 413, US 7,148,382 B2, US 5,761,169, DE 197 08 294 A1, EP 0 057 474, EP 0 047 902 A, EP 0 007 508, DE 600 29 481 T2, EP 0 980 682 B1, EP 0 948 955 B1, EP 1 236 459 B1 und EP 0 173 567 A2 verwiesen, welche im Wege der Verweisung Bestandteil der vorliegenden Anmeldung sind.

Die in diesen Druckschriften angegebenen Phosphinoxide eigenen sich besonders allein oder in Kombination mit dem System "alpha-Diketon/Amin" als Photopolymerisationsinitiatorsystem in den erfindungsgemäßen Zusammensetzungen.

Alternativ können auch Boratsalze, wie sie beispielsweise in US 4,772,530, US 4,954,414, US 4,874,450, US 5,055,372 und US 5,057,393 beschrieben sind, als Photoinitiatoren Verwendung finden, welche im Wege der Verweisung Bestandteil der vorliegenden Anmeldung sind.

Weitere geeignete Photoinitiatoren sind in J.-P. Fouassier, Photoinitiation, Photopolymerization and Photocuring, Hanser Publishers, Munich, Vienna, New York 1995 sowie in J.F. Rabek (Hrsg.), Radiation Curing in Polymer Science and Technology, Vol. II, Elsevier Applied Science, London, New York 1993 beschrieben, welche im Wege der Verweisung Bestandteil der vorliegenden Anmeldung sind.

### Bestandteil (d) - Polymerisationsinhibitoren

Die erfindungsgemäßen dentalen Zusammensetzungen enthalten vorzugsweise einen oder mehrere Inhibitoren, auch Stabilisatoren genannt. Diese werden einer Abdeckmasse zugesetzt, um eine Spontanpolymerisation zu vermeiden. Sie reagieren mit vorzeitig entstehenden Radikalen, die abgefangen werden, verhindern eine vorzeitige Polymerisation und erhöhen die Lagerstabilität der lichthärtbaren dentalen Zusammensetzung. Gängige Inhibitoren sind Phenolderivate wie Hydrochinonmonomethylether (HQME) oder 2,6-Di-tert.butyl-4-methylphenol (BHT). Weitere Inhibitoren wie 2,2 Diphenyl-1-picrylhydrazyl-, Galvinoxyl-, Triphenylmethyl-Radikale, 2,3,6,6,-Tetramethylpiperidinyl-1-oxylradikale (TEMPO) sowie Derivate von TEMPO oder Phenothiazin sowie Derivate dieser Verbindung werden in der EP 0 783 880 B1 beschrieben welche im Wege der Verweisung Bestandteil der vorliegenden Anmeldung sind. Alternative Inhibitoren sind in der DE 101 19 831 A1 oder in der EP 1 563 821 A1 angegeben, welche im Wege der Verweisung Bestandteil der vorliegenden Anmeldung sind.

Eine erfindungsgemäß bevorzugte dentale Zusammensetzung als dentale Abdeckmasse umfasst weiter
(d) ein oder mehrere Polymerisationsinhibitoren zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten.

Die Polymerisationsinhibitoren des Bestandteils (d) sind im Unterschied zu den Molekulargewichtsreglern des Bestandteils (c) nicht zur H-Abstraktion in Allylstellung befähigt.

Sofern dennoch im Einzelfall ein Polymerisationsinhibitor gleichzeitig als ein zur H-Abstraktion in Allylstellung befähigter Molekulargewichtsregler aufgefasst werden kann, wird dieser für die Zwecke des vorliegenden Textes als Molekulargewichtsregler aufgefasst, insbesondere bei quantitativen Betrachtungen.
Die Anwesenheit eines oder mehrerer Polymerisationsinhibitoren des Bestandteils (d), vorzugsweise von BHT und/oder TEMPO, in einer erfindungsgemäßen dentalen Zusammensetzung, insbesondere in den oben genannten bevorzugten Mengen bzw.

Mengenverhältnissen, ist deshalb vorteilhaft, weil die Lagerstabilität einer erfindungsgemäßen dentalen Zusammensetzung signifikant erhöht wird. Dabei wird die hier relevante Wirkung des oder der Molekulargewichtsregler (c) nicht beeinträchtigt.

Eine erfindungsgemäß bevorzugte dentale Zusammensetzung umfasst Bestandteil (d) in einer Menge von 10 bis 2000 ppm, vorzugsweise 50 bis 1.500 ppm, bevorzugt 300 bis 1.000 ppm,
jeweils bezogen auf die Gesamtmasse der dentalen Zusammensetzung.

Werden merklich höhere Mengen an Bestandteil (d) eingesetzt, so wird die Aushärtung der dentalen Zusammensetzung merklich verlangsamt.

In weiteren erfindungsgemäß bevorzugten dentalen Zusammensetzungen ist das Verhältnis der Gesamtmasse des Bestandteils (c) zu der Gesamtmasse des Bestandteils (d) größer ist als 1, vorzugweise größer ist als 2, vorzugsweise größer als 4.

Zusammenfassend gewährleistet eine solche Kombination von Bestandteil (c) und (d), insbesondere in einer der als bevorzugt gekennzeichneten Ausgestaltungen,
- eine sehr gute Lagerstabilität,
- bei Polymerisation der erfindungsgemäßen dentalen Zusammensetzung eine einstellbare / maßgeschneiderte Molekulargewichtsverteilung nach Lichthärtung, und
- ein akzeptables Temperaturmaximum Tₘₐₓ bei der Photopolymerisation der dentalen Zusammensetzung im Mundraum.

Erfindungsgemäße dentale Zusammensetzungen können ferner Füllstoffe enthalten. Als Füllstoffe können anorganische Gläser bzw. Keramiken und/oder organische Füllstoffe verwendet werden. Die Füllstoffe können allein oder als Mischungen eingesetzt werden. Zur Optimierung der Produkteigenschaften können können die Füllstoffe in unterschiedlichen Korngrößen in die Rezepturen eingebracht werden. Die Füllstoffe können eine unimodale oder polymodale, beispielsweise eine bimodale Verteilung aufweisen. Weiterhin können verstärkend wirkende Materialien, wie Glasfasern, Polyamid- oder Kohlenstofffasern eingesetzt werden.

Die dentale Zusammensetzung kann zusätzlich feinteilige Splitter oder Perlpoymerisate enthalten, wobei die Perlpoymerisate Homo- oder Copolymere organischer härtbarer Monomerer sein können.

Ebenfalls bevorzugte erfindungsgemäße dentale Zusammensetzungen sind dadurch gekennzeichnet, dass diese weiter umfassen
(e) einen oder mehrere anorganische Bestandteile, vorzugsweise in einer Gesamtmenge von 5 bis 40 Gew.-%, bevorzugt in einer Gesamtmenge von 10 bis 30 Gew.-%,
jeweils bezogen auf die Gesamtmasse der dentalen Zusammensetzung.

Geeignete anorganische Bestandteile sind beispielsweise amorphe Materialien auf der Basis von Mischoxiden aus SiO₂, ZrO₂ und/oder TiO₂, mikrofeine Füllstoffe wie pyrogene Kieselsäure oder Fällungskieselsäure sowie Makro- oder Mini-Füllstoffe wie Quarz-Glaskeramik oder Glaspulver, Bariumsilikatgläser, Bariumfluorsilikatgläser, Strontiumsilikatgläser, Strontiumborsilikat, Li/Al-Silikatgläser, Bariumgläser, Calciumsilikate, Natriumaluminiumsilikate, Fluoraluminiumsilikatgläser, Oxide von Aluminium oder Silicium, Zeolithe, Apatit, Zirkonsilikate, schwerlösliche Metallsalze wie Bariumsulfat oder Calciumfluorid

Zum besseren Einbau in die Polymermatrix können die Füllstoffe oberflächenmodifiziert sein. Beispielhaft genannt sei die Oberflächenbehandlung der Füllstoffe mit einem Silan. Als Haftvermittler eignet sich besonders das Methacryloxypropyltrimethoxysilan.

Zur Einstellung der Rheologie der erfindungsgemäßen dentalen Abdeckmassen können erfindungsgemäße dentale Zusammensetzungen unterschiedliche Kieselsäuren, bevorzugt pyrogene Kieselsäuren, enthalten. Ferner enthalten erfindungsgemäße dentale Zusammensetzungen vorzugsweise Bariumsulfat zur (weiteren) Erhöhung des optischen Kontrasts einer erfindungsgemäßen dentalen Zusammensetzung zum Zahnfleisch.

In einer bevorzugten erfindungsgemäßen dentalen Zusammensetzung liegt das Massenverhältnis von der Gesamtmenge an photopolymerisierbaren Monomeren ausgewählt aus der Gruppe bestehend aus Acrylaten und Methacrylaten (d.h. der photopolymerisierbaren Monomeren gemäß Komponente (a) wie oben definiert) zu der Gesamtmenge an mikrofeinen Füllstoffen ausgewählt aus der Gruppe bestehend aus pyrogene Kieselsäure und Fällungskieselsäure im Bereich von 3 : 1 bis 4 : 1. Bevorzugt ist auch insoweit der Einsatz pyrogener Kieselsäure. In eigenen Untersuchungen ergaben sich bei Einstellung eines bevorzugten Massenverhältnisses besonders gute rheologische Eigenschaften einer erfindungsgemäßen dentalen Zusammensetzung.

Eine bevorzugte erfindungsgemäße dentale Zusammensetzung ist mit einer dentalen Spritze applizierbar.

Bevorzugt sind dabei nachlauffreie, nichttropfende Spritzen (vorzugsweise Non-Dripping-Technology (NDT^{®}) - Spritzen), da mit diesen eine punktgenaue Applizierung einer erfindungsgemäßen dentalen Zusammensetzung möglich ist und dadurch ein störender Überschuss an erfindungsgemäßer dentaler Zusammensetzung vermieden werden kann.

Eine bevorzugte erfindungsgemäße dentale Zusammensetzung weist bei Applikation im Mund ein rheologisches Anfließverhalten und eine solche Standfestigkeit auf, dass ein Herunterlaufen oder Spreiten der applizierten Zusammensetzung nicht auftritt.

Vorzugsweise ist die Viskosität einer erfindungsgemäßen dentalen Zusammensetzung größer oder gleich 10000 mPas und liegt bevorzugt im Bereich von 10000 mPas bis 80000 mPas.

Bei höheren Viskositätswerten, insbesondere oberhalb von 90000 mPas, wird das Anfließverhalten der Zusammensetzung signifikant schlechter und ist aus einer üblichen dentalen Kanüle nur noch sehr schlecht ausdrückbar.

Bevorzugt ist eine erfindungsgemäße dentale Zusammensetzung, deren Viskosität größer oder gleich 15000 mPas und weiter bevorzugt größer oder gleich 20000 mPas ist.

Ganz besonders bevorzugt sind erfindungsgemäße dentale Zusammensetzungen, deren Viskosität im Bereich von 20000 mPas bis 35000 mPas liegt, insbesondere solche dentalen Zusammensetzungen, die gleichzeitig weitere bevorzugte Materialeigenschaften besitzen, wie sie vorstehend oder nachfolgend angegeben sind.

Die hier angegebenen Viskositäten beziehen sich auf die Messung bei 23°C mit einem Platte-Platte-Rheometer (Durchmesser der Platte: 25 mm) mit einer Scherrate von 10/sec. bei einer Spaltbreite von 1 mm.

Dentale Zusammensetzungen mit einer Viskosität im ganz besonders bevorzugten Bereich von 20000 mPas bis 35000 mPas lassen sich unter Verwendung üblicher dentaler Kanülen besonders komfortabel und in sowohl für den Patienten als auch für den behandelnden Zahnarzt angenehmer Weise auf die Gingiva applizieren, ohne dass es zu unerwünschter Tropfenbildung oder zu einem Wegfließen kommt. Aufgrund der vergleichsweise niedrigen Viskosität deckt die applizierte dentale Zusammensetzung die Gingiva auch in den Interdentalpapillen passgenau ab. Die bevorzugte erfindungsgemäße dentale Zusammensetzung separiert nicht, d.h. die Füllstoffe setzen sich nicht aus der pastösen Phase ab.

Eine bevorzugte erfindungsgemäße dentale Zusammensetzung ist dadurch gekennzeichnet, dass die dentale Zusammensetzung nach Applikation auf Zähne und/oder Zahnfleisch durch Lichthärtung in ein elastisches Material überführbar ist, welches
- rückstandsfrei von den besagten Zähnen oder dem besagten Zahnfleisch abziehbar ist
   und/oder
- einen Elastizitätsmodul < 4000 MPa, vorzugsweise < 2000 MPa, bevorzugt < 1000 MPa besitzt.

Eine bevorzugte erfindungsgemäße dentale Zusammensetzung ist dadurch gekennzeichnet, dass sie nach Applikation auf Zähne und/oder Zahnfleisch durch Lichthärtung in ein dentales elastisches Material zum Einsatz in der Mundhöhle überführbar ist, welches einen Elastizitätsmodul < 4000 MPa, vorzugsweise < 2000 MPa, ganz besonders bevorzugt < 1000 MPa besitzt.

Besonders bevorzugt ist eine erfindungsgemäße photopolymerisierbare dentale Zusammensetzung zur Applikation auf und zum Isolieren und Schützen von Zahnfleisch und/oder Zähnen während einer Zahnbehandlung, umfassend:
(a) ein oder mehrere photopolymerisierbare Monomere ausgewählt aus der Gruppe bestehend aus Acrylaten und Methacrylaten, vorzugsweise der Gruppe der Methacrylate,
(b) ein oder mehrere Photoinitiatoren, vorzugsweise ausgewählt aus der Gruppe bestehend aus alpha-Diketonen, Benzoinalkylethern, Thioxanthonen, Benzophenonen, Acylphosphinoxiden, Acetophenonen, Ketalen, Titanocenen, Boraten und sensibilisierenden Farbstoffen,
(c) ein oder mehrere Molekulargewichtsregler ausgewählt aus der Gruppe bestehend aus α-Terpinen und γ-Terpinen (dies sind Verbindungen, die mit einem Radikal eines Monomers des Bestandteils (a) unter Abstraktion eines H-Radikals aus dem Molekulargewichtsregler in Allylposition umsetzbar sind),
sowie gegebenenfalls ein oder mehrere weitere Additive, wobei
die dentale Zusammensetzung nach Applikation auf Zähne und/oder Zahnfleisch durch Lichthärtung in ein elastisches Material überführbar ist, welches einen Elastizitätsmodul < 1000 MPa besitzt.

Sämtliche im vorliegenden Text angegebenen bevorzugten Ausgestaltungen betreffen insbesondere auch diese besonders bevorzugte Zusammensetzung.

Vorzugsweise ist die erfindungsgemäße dentale Zusammensetzung durch Lichthärtung nicht in ein elastisches Material überführbar, welches einen Elastizitätsmodul > 2000MPa besitzt, besonders bevorzugt nicht in ein elastisches Material überführbar, welches einen Elastizitätsmodul > 1000 MPa besitzt.

Gemäß dieser bevorzugten Ausgestaltung ist es auch durch intensive Bestrahlung mit Licht nicht möglich, die dentale Zusammensetzung in ein elastisches Material mit vergleichsweise geringer Elastizität zu überführen.

Sämtliche im vorliegenden Text angegebenen bevorzugten Ausgestaltungen betreffen insbesondere auch diese besonders bevorzugte Zusammensetzung.

Der Elastizitätsmodul (Biegemodul) wird dabei aus Biegeversuchen gemäß der Norm ISO 4049 bestimmt, wobei Mittelwerte aus 5 Einzelmessungen (3-Punkt-Biegeversuch; Flach-/Streifenprobe) gebildet werden.

Besonders bevorzugte erfindungsgemäße dentale Zusammensetzungen besitzen eine Durchhärtetiefe (Polymerisationstiefe, DHT) im Bereich von 1,2 bis 2,2 mm, vorzugsweise 1,5 bis 1,8 mm. Die Durchhärtetiefe wird dabei analog der Norm ISO 4049 mit einer Belichtungszeit von 10 sec. bestimmt, wobei Mittelwerte aus 3 Einzelmessungen gebildet werden. Eine Durchhärtetiefe im bevorzugten Bereich gewährleistet einen schnell erreichbaren Schutz der Gingiva.

Besonders bevorzugte erfindungsgemäße dentale Zusammensetzungen sind durch Lichthärtung in ein elastisches Material überführbar, welches eine Biegefestigkeit von weniger als 50 MPa besitzt, ganz besonders bevorzugt von weniger als 47 MPa. Die Biegefestigkeit wird dabei entsprechend der Norm ISO 4049 bestimmt, wobei Mittelwerte aus 5 Einzelmessungen gebildet werden. Besonders bevorzugte erfindungsgemäße dentale Zusammensetzungen sind daher gemäß ISO 4049 nicht als Restaurationsmaterial geeignet.Vorzugsweise ist die erfindungsgemäße dentale Zusammensetzung durch Lichthärtung nicht in ein elastisches Material überführbar, welches eine Biegefestigkeit von größer als 50 MPa besitzt, besonders bevorzugt ist die erfindungsgemäße dentale Zusammensetzung durch Lichthärtung nicht in ein elastisches Material überführbar, welches eine Biegefestigkeit von größer als 47 MPa besitzt.

Kurz zusammengefasst weist eine erfindungsgemäße dentale Zusammensetzung, insbesondere in einer der bevorzugten bzw. besonders bevorzugten Ausgestaltungen, folgende Eigenschaften und Vorteile auf:
- eine erfindungsgemäße dentale Zusammensetzung deckt die Gingiva bis in die Interdentalräume optimal ab, ohne dabei wegzufließen (dies gilt insbesondere bei Einstellung einer vorstehend als bevorzugt angegebenen Viskosität);
- eine erfindungsgemäße dentale Zusammensetzung zeigt ein sehr gutes Anfließverhalten und eine gute und einfache Entfernbarkeit nach dem Aushärten;
- die freigesetzte Wärmemenge und die damit verbundene Temperaturerhöhung (bis zum Temperaturmaximum Tₘₐₓ) während der Lichthärtung einer erfindungsgemäßen dentalen Zusammensetzung ist gering bis moderat und wird von vielen Personen als nicht störend und sogar als angenehm empfunden;
- nach Aushärtung verrutscht eine erfindungsgemäße dentale Zusammensetzung während der Behandlung nicht und lässt sich nach der Behandlung in einem Stück entfernen;
- nach Lichthärtung besitzt eine erfindungsgemäße dentale Zusammensetzung einen besonders niedrigen Elastizitätsmodul, also eine besonders hohe Elastizität und Nachgiebigkeit (siehe dazu oben), die sie von chemisch ähnlich zusammengesetzten Zusammensetzungen aus dem Stand der Technik deutlich unterscheidet,
- nach Lichthärtung besitzt eine erfindungsgemäße dentale Zusammensetzung vorzugsweise eine besonders niedrige Biegefestigkeit (siehe oben)
- die rheologischen Eigenschaften der erfindungsgemäßen dentalen Zusammensetzung sind vorzugsweise durch das Vorhandensein mikrofeiner Füllstoffe (vorzugsweise pyrogener Kieselsäure) in geeigneter Menge (siehe dazu oben) für den erfindungsgemäßen Einsatzzweck optimiert.
- die Viskosität der erfindungsgemäßen dentalen Zusammensetzung ist vorzugsweise so eingestellt (siehe dazu oben), dass ihre Applikation auf die Gingiva unter Verwendung üblicher dentaler Kanülen besonders komfortabel und sowohl für den Patienten als auch für den behandelnden Zahnarzt angenehm ist, ohne dass es zu unerwünschter Tropfenbildung oder zu einem Wegfließen kommt.

Vorzugsweise sind die Anteile der Bestandteile einer erfindungsgemäßen dentalen Zusammensetzung so gewählt, dass die vollständige Lichtaushärtung innerhalb von 20 Sekunden, bevorzugt innerhalb von 10 Sekunden, pro Lichtführungsbreite erfolgt, wenn die applizierte Schicht der dentalen Zusammensetzung bei einer Dicke von 1,5 mm mit einer Lampe mit 1000 mW/cm² eines handelsüblichen dentalen Polymerisationsgerätes bestrahlt wird.

In einer bevorzugten Ausführungsform enthält eine erfindungsgemäße dentale Zusammensetzung keine Weichmacher und/oder keine Lösungmittel, d.h. keine nicht reaktiven organischen Bestandteile. Vorzugsweise enthält eine erfindungsgemäße dentale Zusammensetzung kein Cetylalkohol.

UV-Absorber, die beispielsweise durch ihre konjugierten Doppelbindungssytemen und aromatischen Ringe befähigt sind, UV Strahlung zu absorbieren, können gegebenenfalls Bestandteil einer erfindungsgemäßen dentalen Zusammensetzungen sein. Beispiele an UV-Absorbern sind 2-Hydroxy-4-methoxybenzophenon, Salicylsäurephenylester oder 3-(2'-Hydroxy-5'-methylphenyl)-benzotriazol.

Für eine bessere Applikationskontrolle ist es vorteilhaft, die erfindungsgemäßen dentalen Zusammensetzungen in unterschiedlichen Farbtönen bereitzustellen, welche sich vorzugsweise merklich von der Farbe des Zahnfleischs und/oder der Zähne unterscheidet. Zu diesem Zweck werden vorzugsweise anorganische Farbstoffe und/oder organische Pigmente eingesetzt, um eine deutliche Kontrastgebung zur besseren Applikationskontrolle herzustellen.

Weitere optionale Bestandteile sind Aromastoffe.

Die Erfindung betrifft ferner die Verwendung von α-Terpinen oder γ-Terpinen, welche Verbindungen mit zwei oder mehr Doppelbindungen sind, die durch Abstraktion eines Allyl-H-Atoms ein delokalisiertes Elektronensystem ausbilden können, das sich über 5 oder mehr C-Atome der Verbindung erstreckt,
und insbesondere. Diene sind, die durch Abstraktion eines Allyl-H-Atoms ein delokalisiertes Elektronensystem ausbilden können, das sich über 5 C-Atome erstreckt,
als Molekulargewichtsregler bei der Polymerisation von Monomeren ausgewählt aus der Gruppe bestehend aus Acrylaten und Methacrylaten, vorzugsweise aus der Gruppe der Methacrylate,
zur Herstellung einer dentalen Abdeckmasse.

Für die erfindungsgemäßen Kits und Verfahren gelten die obigen Angaben zu bevorzugten bzw. besonders bevorzugten erfindungsgemäßen Zusammensetzungen entsprechend.

Für die erfindungsgemäßen Kits und Verfahren gelten die obigen Angaben zu bevorzugten bzw. besonders bevorzugten erfindungsgemäßen Zusammensetzungen entsprechend, insbesondere in einer der als bevorzugt angegebenen Ausgestaltungen.

Ferner betrifft die Erfindung ein Kit umfassend:
- eine erfindungsgemäße dentale Zusammensetzung, und
- ein Mittel zum Behandeln eines Zahnes, welches bei Kontakt mit einem Zahn diesen und/oder bei Kontakt mit dem Zahnfleisch dieses verändert.

Das Mittel zum Behandeln eines Zahnes ist eines, welches unterschiedslos sowohl auf einen zu behandelnden Zahn als auch auf nicht zu behandelnde Nachbarzähne und/oder das Zahnfleisch einwirkt, wenn es in Kontakt damit tritt. Deshalb muss es von Nachbarzähnen und/ oder Zahnfleisch isoliert werden.

Bevorzugt ist ein erfindungsgemäßes Kit, wobei das Mittel zum Behandeln eines Zahnes ein Mittel zur dentalen Zahnbleichung ist.

Offenbart wird ebenfalls ein Verfahren zum Herstellen einer Schutzschicht zum Abdecken von Zahnfleisch und/oder Zähnen und zum Isolieren des Zahnfleisches bzw. der Zähne gegenüber benachbarten Zahnpartien, mit folgenden Schritten:
- Herstellen einer erfindungsgemäßen photopolymerisierbaren dentalen Zusammensetzung durch Vermischen der Bestandteile (a), (b) und (c) sowie optional Bestandteil (d) und gegebenenfalls weiterer Bestandteile,
- Bestrahlen der hergestellten Zusammensetzung mit Licht, so dass der Bestandteil (b) die Photopolymerisation initiiert.

Offenbart wird ferner ein Verfahren zum kosmetischen Behandeln eines oder mehrerer Zähne, mit folgenden Schritten:
(i) Identifizieren eines oder mehrerer zu behandelnder Zähne,
(ii) Applizieren einer erfindungsgemäßen photopolymerisierbaren dentalen Zusammensetzung auf das Zahnfleisch oder einen oder mehrere Zähne in Nachbarschaft zu dem oder den zu behandelnden Zähnen,
(iii) Lichthärten der applizierten photopolymerisierbaren dentalen Zusammensetzung,
(iv) Kosmetisches Behandeln des oder der gemäß Schritt (i) identifizierten Zähne.

Das Applizieren einer erfindungsgemäßen photopolymerisierbaren dentalen Zusammensetzung als dentale Abdeckmasse wird vorzugsweise dergestalt ausgeführt, dass die erfindungsgemäße photopolymerisierbare dentale Zusammensetzung etwa 0,5 mm bis 1 mm des Zahnhalses des zu behandelnden Zahnes bedeckt. Wenn etwa 0,5 mm der cervikaten Zahnhartsubstanz des zu behandelnden Zahnes zusätzlich mit der erfindungsgemäßen photopolymerisierbaren dentalen Zusammensetzung bedeckt werden, ist nach deren Aushärtung ein besonders effektiver Schutz der Gingiva gewährleistet und es wird ein besonders dichter Abschluss am Zahnhalssaum zur Gingiva erreicht. Vorzugsweise sollte die erfindungsgemäße photopolymerisierbare dentale Zusammensetzung ca. 3 - 10 mm weit auf die benachbarte Gingiva erstrecken.

Eine kosmetische (ästhetische) Behandlung ist vorzugsweise eine Behandlung zur Zahnaufhellung (Bleaching), zum Aufbringen von Veneers (keramische Verblendschalen, die auf den Zahn aufgeklebt werden) und das Aufbringen von Zahnschmuck (beispielsweise falsche Diamanten, (Strass)Steinchen, Bildchen, Goldplättchen oder - folien) auf einen Zahn (auch bekannt als Dazzler, Twinkles, Zahn-Tattoos usw.). Bevorzugt ist die kosmetische Behandlung eine Zahnaufhellung.

Die vorliegende Erfindung betrifft auch die Verwendung einer erfindungsgemäßen dentalen Zusammensetzung zum
- Herstellen einer Schutzschicht zum Abdecken von Zahnfleisch und/oder Zähnen und zum Isolieren des Zahnfleisches bzw. der Zähne gegenüber benachbarten Zahnpartien
   und/oder
- in einem Verfahren zum kosmetischen Behandeln einer oder mehrerer Zähne.

Es sind hier ebenfalls die zu den oben beschriebenen Verfahren angegebenen bevorzugten Ausgestaltungen bevorzugt.

Anhand der folgenden Beispiele wird die Erfindung weiter erläutert. Sofern nicht anders angegeben, beziehen sich alle Angaben auf das Gewicht. Es werden dabei folgende branchenübliche Abkürzungen verwendet:
BHT = 2,6-Di-tert.butyl-4-methylphenol
UDMA = Urethandimethacrylat (7,7,9-Trimethyl-4,13-dioxo-5,12-diazahexadecan-1,16-dioxydimethacrylat)
MDP = 10-(Meth)acryloyloxydecyldihydrogenphosphat
CC = Campherchinon
DABE = Ethyl-*p*-*N,N*-dimethylaminobenzoat
Bis-GMA = Bisphenol-A-Glycidyl-Methacrylat
TEGDMA = Triethylenglykoldimethacrylat
4-META = 4-Methacrylolyoxyethyloxycarbonyl-phthalsäureanhydrid (4-(Meth)acryloxyethyltrimellithsäureanhydrid)
TCD-di-HEMA = Bis(methacrylolyoxymethyl)tricyclo[5.2.10^{2,6}]decan
HEDMA = 1,6-Hexandioldimethacrylat
GlyDMA-Phosphat = 1,2- und 1,3-Glycerindimethacrylatphosphat
HEMA = Hydroxyethylmethacrylat

### Beschreibung der Methoden:

### Temperaturmaximum (Tₘₐₓ)

Die Wärmeentwicklung während der Polymerisation wurde mit einer relativen Messmethode bestimmt: ein Thermofühler Typ L (Fe-CuNi) der Fa. Reckmann wird in eine Halterung aus Polyethylen so eingebettet, dass 3 mm des Fühlers herausragen. Dieses freie Ende des Thermofühlers wird mittig in ein Polyethylenröhrchen von 3 mm Innendurchmesser und 5 mm Höhe platziert. Das Röhrchen wird mit Material randvoll gefüllt. Auf das gefüllte Röhrchen wird eine farblose klare Acetatfolie gelegt, so dass evtl. Materialüberschüsse verpresst werden. Um die Polymerisation auszulösen, wird eine dentale LED-Lampe mit 1000 mW/cm² Lichtleistung direkt auf die Acetatfolie aufgesetzt und für 10 sec eingeschaltet. Die aus der Temperaturentwicklung resultierende Spannungsänderung des Thermoelementes wird an einem xy-Schreiber L 250-2 der Fa. Linseis bei einer Empfindlichkeit von 2 mV aufgezeichnet.

Die Messung erfolgt in einem klimatisierten Raum bei 23°C und 50% relative Luftfeuchte unter gelbem Schutzlicht, um eine vorzeitige Polymerisation der Materialien zu vermeiden. Jede Einzelmessung wird erst dann gestartet (belichtet), wenn nach dem Auflegen der Acetatfolie wieder eine konstante Temperatur erreicht ist und der Messschreiber auf die Nulllinie justiert wurde. Gemessen wird der Maximalausschlag des Schreibers in Skalenteilen (0 - 100 Skt.). Der Klarheit halber sei angemerkt, dass die Skalenteile (Skt.) zwar mit der Temperatur korrelieren (d.h. innerhalb einer Messreihe entspricht ein höherer Skt.-Wert einer höheren Temperatur), jedoch zahlenmäßig nicht mit °C-Werten übereinstimmen. Die angegebenen Messwerte sind jeweils die Mittelwerte aus 3 Einzelmessungen.

Tₘₐₓ sollte vorzugsweise im Bereich < 50 Skt. liegen, da in einem solchen Fall die Wärmemenge und die Temperatur für den betroffenen Personen als akzeptabel bzw. sogar als angenehm empfunden wurde.

### Durchhärtetiefe (DHT)

Die Durchhärtetiefe wurde analog der Norm ISO 4049 mit einer Belichtungszeit von 10 sec. bestimmt. Die angegebenen Messwerte sind jeweils die Mittelwerte aus 3 Einzelmessungen.

### Biegefestigkeit (BF) und Elastizitätsmodul

Die Biegefestigkeit wurde entsprechend der Norm ISO 4049 an einer Materialprüfmaschine der Fa. Zwick bestimmt. Die angegebenen Messwerte sind jeweils die Mittelwerte aus 5 Einzelmessungen. Der Elastizitätsmodul (Biegemodul) ergibt sich in bekannter Weise aus Biegeversuchen gemäß ISO 4049 (3-Punkt-Biegeversuch, Flach-Streifenprobe).

**Tabelle 1: Zusammensetzungen mit γ-Terpinen als Molekulargewichtsregler:**

| Bei den Mengenangaben handelt es sich um Gewichtsteile | | | | | | |
|---|---|---|---|---|---|---|
| | Referenz | Exp. 1 | Exp. 2 | Exp. 3 | Exp. 4 | Exp. 5 |
| ethoxyliertes Bisphenol A dimethacrylat | 12,117 | 12,117 | 12,117 | 12,117 | 12,117 | 12,117 |
| UDMA | 60,575 | 60,575 | 60,575 | 60,574 | 60,575 | 60,575 |
| MDP | 2,000 | 2,000 | 2,000 | 1,999 | 2,000 | 2,000 |
| CC | 0,146 | 0,146 | 0,146 | 0,146 | 0,146 | 0,146 |
| DABE | 0,219 | 0,219 | 0,219 | 0,219 | 0,219 | 0,219 |
| Pfefferminzaroma | 1,010 | 1,010 | 1,010 | 1,010 | 1,010 | 1,010 |
| Bariumsulfat | 2,999 | 2,999 | 2,999 | 2,999 | 2,999 | 2,999 |
| pyrogene Kieselsäure | 20,005 | 20,005 | 20,005 | 20,005 | 20,005 | 20,005 |
| Blauer Farbstoff | 0,501 | 0,501 | 0,501 | 0,501 | 0,501 | 0,501 |
| BHT | 0,073 | 0,073 | 0,073 | 0,073 | 0,073 | 0,073 |
| γ-Terpinen | | 0,094 | 0,213 | 0,358 | 0,520 | 0,651 |
| | | | | | | |
| Tₘₐₓ [Skt.] | 64 | 57 | 49 | 43 | 37 | 34 |
| DHT [mm] | 2,03 | 1,91 | 1,72 | 1,63 | 1,45 | 1,32 |

Als pyrogene Kieselsäure wurde Aerosil^{®} DT4 eingesetzt.

Die Biegefestigkeit (BF) der Abdeckmasse gemäß Exp. 3 betrug 17,5 MPa; die Haftkraft betrug 2,8 MPa, gemessen an Rinderschmelz nach der in ISO CD 29022 beschriebenen Methode.

Die Viskosität der Abdeckmasse gemäß Exp. 3 wurde bei 23°C mit einem Platte-Platte-Rheometer (Durchmesser der Platte: 25 mm) mit einer Scherrate von 10/sec. bei einer Spaltbreite von 1 mm bestimmt; die Viskosität betrug 27000 mPa s

Der Elastizitätsmodul der Abdeckmasse gemäß Exp. 3 (separate Umsetzung) betrug nach Lichthärtung 195 MPa.

**Tabelle 2: Weitere Ausführungsbeispiele**

| Bei den Mengenangaben handelt es sich um Gewichtsteile | | | | | | | |
|---|---|---|---|---|---|---|---|
| | | Exp. 6 | Exp. 7 | Exp. 8 | Exp. 9 | Exp. 10 | Exp. 11 |
| Monomere | UDMA | 49,7 | 49,7 | 60,7 | 10,0 | 10,0 | 10,0 |
| | ethoxyliertes Bisphenol A dimethacrylat | 15,0 | 15,0 | 12,0 | 8,0 | 8,0 | 8,0 |
| | TCD-di-HEMA | | | | 15,0 | 15,0 | 15,0 |
| | TEGDMA | 7,3 | 7,3 | | 7,0 | 7,0 | 7,0 |
| | Bis-GMA | | | | 17,0 | 17,0 | 17,0 |
| | HEDMA | | | | 12,0 | 12,0 | 12,0 |
| Säuregruppenhaltige Monomere | GlyDMA-Phosphat | 2,0 | | | | | |
| | MDP | | 2,0 | | | | |
| | Di-HEMA-Phosphat | | | 2,0 | 1,0 | 1,0 | 1,0 |
| | 4-META | | | | 1,0 | 1,0 | 1,0 |
| | CC | 0,15 | 0,15 | 0,15 | 0,10 | 0,10 | 0,10 |
| | DABE | 0,22 | 0,22 | 0,22 | 0,14 | 0,14 | 0,14 |
| | Bariumsulfat | 3,0 | 3,0 | 3,0 | 1,7 | 1,7 | 1,7 |
| | Glaskeramik | 5,7 | 5,7 | | 11,0 | 11,0 | 11,0 |
| | pyrogene Kieselsäure | 15,0 | 15,0 | 20,0 | 15,0 | 15,0 | 15,0 |
| | Farbstoff | 0,50 | 0,50 | 0,50 | 0,50 | 0,50 | 0,50 |
| | BHT | 0,08 | 0,08 | 0,08 | 0,06 | 0,06 | 0,06 |
| | Pfefferminzaroma | 1,00 | 1,00 | 1,00 | 1,00 | 1,00 | 1,00 |

| | | Exp. 6 | Exp. 7 | Exp. 8 | Exp. 9 | Exp. 10 | Exp. 11 |
|---|---|---|---|---|---|---|---|
| Regler | *γ*-Terpinen | 0,35 | 0,35 | 0,35 | 0,50 | | |
| | 2,4-Diphenyl-4-methylpenten (nicht erfindungsgemäß) | | | | | 0,50 | |
| | *α-*Linolensäure nicht erfindungsgemäß) | | | | | | 0,50 |
| | | | | | | | |
| | Tₘₐₓ [Skt.] | 44 | 43 | 45 | 41 | 52 | 49 |

**Tabelle 3: Cetylalkohol (nicht erfindungsgemäß)**

| Bei den Mengenangaben handelt es sich um Gewichtsteile | | | | |
|---|---|---|---|---|
| | Referenz | Exp. 3-1 | Exp. 3-2 | Exp. 3-3 |
| ethoxyliertes Bisphenol A dimethacrylat | 12,117 | 12,117 | 12,117 | 12,117 |
| UDMA | 60,575 | 60,574 | 60,575 | 60,575 |
| MDP | 2,000 | 1,999 | 2,000 | 2,000 |
| Di-HEMA-Phosphat | 0 | 0 | 0 | 0 |
| CC | 0,146 | 0,146 | 0,146 | 0,146 |
| DABE | 0,219 | 0,219 | 0,219 | 0,219 |
| Pfefferminzaroma | 1,010 | 1,010 | 1,010 | 1,010 |
| Bariumsulfat | 2,999 | 2,999 | 2,999 | 2,999 |
| pyrogene Kieselsäure | 20,005 | 20,005 | 20,005 | 20,005 |
| Farbstoff | 0,501 | 0,501 | 0,501 | 0,501 |
| BHT | 0,073 | 0,073 | 0,073 | 0,073 |
| Cetylalkohol | | 0,099 | 0,177 | 0,500 |
| | | | | |
| Tₘₐₓ [Skt.] | 64 | 61 | 62,5 | 57,5 |

**Tabelle 4: BHT**

| Bei den Mengenangaben handelt es sich um Gewichtsteile | | | | |
|---|---|---|---|---|
| | Referenz | Exp. 4-1 | Exp. 4-2 | Exp. 4-3 |
| ethoxyliertes Bisphenol A dimethacrylat | 12,2 | 12,2 | 12,2 | 12,2 |
| UDMA | 60,6 | 60,6 | 60,6 | 60,6 |
| MDP | 2,0 | 2,0 | 2,0 | 2,0 |
| CC | 0,146 | 0,146 | 0,146 | 0,146 |
| DABE | 0,219 | 0,219 | 0,219 | 0,219 |
| Pfefferminzaroma | 1,00 | 1,00 | 1,00 | 1,00 |
| Bariumsulfat | 3,00 | 3,00 | 3,00 | 3,00 |
| pyrogene Kieselsäure | 20,0 | 20,0 | 20,0 | 20,0 |
| Farbstoff | 0,50 | 0,50 | 0,50 | 0,50 |
| BHT | | 0,07 | 0,21 | 0,35 |
| | | | | |
| Tₘₐₓ [Skt.] | 64 | 61 | 57 | 55 |

**Tabelle 5: γ-Terpinen ohne BHT**

| Bei den Mengenangaben handelt es sich um Gewichtsteile | | | | | | |
|---|---|---|---|---|---|---|
| | Referenz | Exp. 5-1 | Exp. 5-2 | Exp. 5-3 | Exp. 5-4 | Exp. 5-5 |
| ethoxyliertes Bisphenol A dimethacrylat | 12,2 | 12,2 | 12,2 | 12,2 | 12,2 | 12,2 |
| UDMA | 60,6 | 60,6 | 60,6 | 60,6 | 60,6 | 60,6 |
| MDP | 2,0 | 2,0 | 2,0 | 2,0 | 2,0 | 2,0 |
| CC | 0,146 | 0,146 | 0,146 | 0,146 | 0,146 | 0,146 |
| DABE | 0,219 | 0,219 | 0,219 | 0,219 | 0,219 | 0,219 |
| Pfefferminzaroma | 1,00 | 1,00 | 1,00 | 1,00 | 1,00 | 1,00 |

| | Referenz | Exp. 5-1 | Exp. 5-2 | Exp. 5-3 | Exp. 5-4 | Exp. 5-5 |
|---|---|---|---|---|---|---|
| Bariumsulfat | 3,00 | 3,00 | 3,00 | 3,00 | 3,00 | 3,00 |
| pyrogene Kieselsäure | 20,0 | 20,0 | 20,0 | 20,0 | 20,0 | 20,0 |
| Farbstoff | 0,50 | 0,50 | 0,50 | 0,50 | 0,50 | 0,50 |
| γ-Terpinen | | 0,07 | 0,21 | 0,35 | 0,49 | 0,63 |
| | | | | | | |
| Tₘₐₓ [Skt.] | 64 | 60 | 50 | 44 | 39 | 34 |

## Patentansprüche

1. Photopolymerisierbare dentale Zusammensetzung als dentale Abdeckmasse zur Applikation auf und zum Isolieren und Schützen von Zahnfleisch und/oder Zähnen während einer Zahnbehandlung, umfassend:
(a) ein oder mehrere photopolymerisierbare Monomere ausgewählt aus der Gruppe bestehend aus Acrylaten und Methacrylaten,
(b) ein oder mehrere Photoinitiatoren,
(c) ein oder mehrere Molekulargewichtsregler ausgewählt aus der Gruppe bestehend aus *α*-Terpinen und *γ*-Terpinen
sowie gegebenenfalls ein oder mehrere weitere Additive.

2. Dentale Zusammensetzung nach Anspruch 1, umfassend
(a) ein oder mehrere photopolymerisierbare Monomere ausgewählt aus der Gruppe der Methacrylate,
und/oder
(b) ein oder mehrere Photoinitiatoren ausgewählt aus der Gruppe bestehend aus alpha-Diketonen, Benzoinalkylethern, Thioxanthonen, Benzophenonen, Acylphosphinoxiden, Acetophenonen, Ketalen, Titanocenen, Boraten und sensibilisierenden Farbstoffen,

3. Dentale Zusammensetzung nach einem der vorangehenden Ansprüche, umfassend
Bestandteil (a) in einer Menge von 50 bis 90 Gew.-%, vorzugsweise in einer Menge von 60 bis 80 Gew.-%,
Bestandteil (b) in einer Menge von 0,01 bis 1 Gew.-%, vorzugsweise von 0,05 bis 0,5 Gew.-%, bevorzugt von 0,1 bis 0,3 Gew.-%, und
Bestandteil (c) in einer Menge von 0,075 bis 7,5 Gew.-%, vorzugsweise von 0,125 bis 3 Gew.-%, bevorzugt von 0,2 bis 1,5 Gew.-%,
jeweils bezogen auf die Gesamtmasse der dentalen Zusammensetzung.

4. Dentale Zusammensetzung nach einem der vorangehenden Ansprüche, wobei Bestandteil (a) umfasst:
(a) (i) ein oder mehrere Dimethacrylat-Monomere
und/oder
(a) (ii) ein oder mehrere säuregruppenhaltige Monomere,
und wobei Bestandteil (a) vorzugsweise sowohl Bestandteil (a) (i) als auch (a) (ii) umfasst,
wobei vorzugsweiseBestandteil (a) (i) ein oder mehrere Dimethacrylat-Monomere enthält gewählt aus der Gruppe bestehend aus Ethylenglykoldimethacrylat (EGDMA), 1,6-Hexandioldimethacrylat (HEDMA), Triethylenglykoldimethacrylat (TEGDMA), 1,12-Dodecandioldimethacrylat (DODMA), ethoxyliertes Bisphenol-A-dimethacrylat, Polyethylenglykoldimethacrylat (PEGDMA), 7,7,9-Trimethyl-4,13-dioxo-5,12-diazahexadecan-1,16-dioxydimethacrylat (UDMA), Butandioldimethacrylat, Tetraethylenglykoldimethacrylat, Neopentylglykoldimethacrylat, 2-Hydroxypropyl-1,3-dimethacrylat, 3-Hydroxypropyl-1,2-dimethacrylat, Pentaerythritoldimethacrylat, Glycerindimethacrylat, Bisphenol-A-Glycidyl-Methacrylat (Bis-GMA) und Dimethacrylaten des Dihydroxymethyltricyclo[5.2.1.0^{2,6}]decans
und/oder
wobei vorzugsweise Bestandteil (a) (ii) ein oder mehrere säuregruppenhaltige Monomere enthält, welche eine Carbonsäure-, eine Phosphorsäure-, eine Phosphonsäure-, eine Sulfonsäure- und/oder eine Thiophosphorsäurefunktion aufweisen, vorzugsweise eine Phosphorsäurefunktion.

5. Dentale Zusammensetzung nach Anspruch 4, **dadurch gekennzeichnet, dass**
Bestandteil (a) (ii)
- ein oder mehrere eine Phosphorsäuregruppe enthaltende Monomere enthält, welche ausgewählt sind aus der Gruppe bestehend aus 2-(Meth)acryloyloxyethyldihydrogenphosphat, Bis[2-(meth)acryloyloxyethyl]-hydrogenphosphat, 2-(Meth)acryloyloxyethylphenylhydrogenphosphat, 6-(Meth)-acryloyloxyhexyldihydrogenphosphat, 10-(Meth)acryloyloxydecyldihydrogenphosphat (MDP), 6-(Meth)-acryloyloxyhexylphenylhydrogenphosphat, 10-(Meth)acryloyloxydecyldihydrogenphosphat, 1,3-Di-(meth)acryloyloxypropan-2-dihydrogenphosphat, 1,3-Di-(meth)acryloyloxypropan-2-phenylhydrogenphosphat und Bis[5-(2-(meth)acryloyloxyethoxycarbonyl)heptyl]hydrogenphosphat,
und/oder
- ein oder mehrere eine Carbonsäuregruppe enthaltende Monomere enthält, welche ausgewählt sind aus der Gruppe bestehend aus 4-(Meth)acryloxyethyltrimellithsäure (4-MET), 4-(Meth)acryloxyethyltrimellithsäureanhydrid (4-META), 4-(Meth)acryloxydecyltrimellithsäure, 4-(Meth)acryloxydecyltrimellithsäureanhydrid, 11-(Meth)acryloyloxy-1,1-undecandicarbonsäure, 1,4-Di(meth)acryloyloxypyromellithsäure, 2-(Meth)acryloyloxyethylmaleinsäure, 2-(Meth)acryloyloxyethylphthalsäure und 2-(Meth)acryloyloxyethylhexahydrophthalsäure.

6. Dentale Zusammensetzung nach einem der Ansprüche 4 bis 5, umfassend Bestandteil (a) (i) in einer Menge von 42 bis 89,5 Gew.-%, vorzugsweise in einer Menge von 56 bis 79 Gew.-%,
und/oder
Bestandteil (a) (ii) in einer Menge von 0,5 bis 8 Gew.-%, vorzugsweise in einer Menge von 1 bis 4 Gew.-%,
jeweils bezogen auf die Gesamtmasse der dentalen Zusammensetzung.

7. Dentale Zusammensetzung nach einem der vorangehenden Ansprüche, weiter umfassend
(d) ein oder mehrere Polymerisationsinhibitoren zur Erhöhung der Lagerstabilität der Zusammensetzung, vorzugsweise ausgewählt aus der Gruppe bestehend aus Hydrochinonmonomethylether (HQME), Phenolen, dabei vorzugsweise 2,6-Di-tert.butyl-4-methylphenol (BHT) und tert.-Butylhydroxyanisol (BHA), 2,2-Diphenyl-1-picrylhydrazyl-Radikalen, Galvinoxyl-Radikalen, Triphenylmethyl-Radikalen, 2,3,6,6,-Tetramethylpiperidinyl-1-oxylradikal (TEMPO) sowie dessen Derivaten und Phenothiazin sowie dessen Derivaten,
vorzugsweise umfassend
Bestandteil (d) in einer Menge von 10 bis 2000 ppm, vorzugsweise 50 bis 1500 ppm, bevorzugt 300 bis 1000 ppm,
jeweils bezogen auf die Gesamtmasse der dentalen Zusammensetzung,
wobei bevorzugt das Verhältnis der Gesamtmasse des Bestandteils (c) zu der Gesamtmasse des Bestandteils (d) größer ist als 1, vorzugweise größer ist als 2, vorzugsweise größer als 4.

8. Dentale Zusammensetzung nach einem der vorangehenden Ansprüche, wobei die dentale Zusammensetzung mit einer dentalen Spritze applizierbar ist
und/oder
bei Applikation im Mund ein rheologisches Anfließverhalten und eine solche Standfestigkeit aufweist, dass ein Herunterlaufen oder Spreiten der applizierten Zusammensetzung nicht auftritt.

9. Dentale Zusammensetzung nach einem der vorangehenden Ansprüche, **dadurch gekennzeichnet, dass** die dentale Zusammensetzung nach Applikation auf Zähne und/oder Zahnfleisch durch Lichthärtung in ein elastisches Material überführbar ist,
- welches rückstandsfrei von den besagten Zähnen oder dem besagten Zahnfleisch abziehbar ist
und/oder
- einen Elastizitätsmodul < 4000 MPa, vorzugsweise < 2000 MPa, bevorzugt < 1000 MPa besitzt.

10. Dentale Zusammensetzung nach einem der vorangehenden Ansprüche, **dadurch gekennzeichnet, dass** die Viskosität der dentalen Zusammensetzung größer oder gleich 10000 mPas ist, vorzugsweise größer oder gleich 15000 mPas und weiter bevorzugt größer oder gleich 20000 mPas und/oder die Viskosität der dentalen Zusammensetzung im Bereich von 10000 mPas bis 80000 mPas liegt, bevorzugt im Bereich von 20000 mPas bis 35000 mPas.

11. Dentale Zusammensetzung nach einem der vorangehenden Ansprüche, **dadurch gekennzeichnet, dass** die dentale Zusammensetzung durch Lichthärtung nicht in ein elastisches Material überführbar ist, welches einen Elastizitätsmodul > 2000MPa besitzt, vorzugsweise nicht in ein elastisches Material überführbar ist, welches einen Elastizitätsmodul > 1000 MPa besitzt.

12. Dentale Zusammensetzung nach einem der vorangehenden Ansprüche, **dadurch gekennzeichnet, dass** die dentale Zusammensetzung eine Durchhärtetiefe im Bereich von 1,2 bis 2,2 mm, vorzugsweise 1,5 bis 1,8 mm, besitzt.

13. Dentale Zusammensetzung nach einem der vorangehenden Ansprüche, **dadurch gekennzeichnet, dass** die dentale Zusammensetzung
durch Lichthärtung in ein elastisches Material überführbar ist, welches eine Biegefestigkeit von weniger als 50 MPa, vorzugsweise von weniger als 47 MPa, besitzt und/oder
durch Lichthärtung nicht in ein elastisches Material überführbar ist, welches eine Biegefestigkeit von größer als 50 MPa besitzt, besonders bevorzugt durch Lichthärtung nicht in ein elastisches Material überführbar ist, welches eine Biegefestigkeit von größer als 47 MPa besitzt.

14. Dentale Zusammensetzung nach einem der vorangehenden Ansprüche, weiter umfassend
(e) einen oder mehrere anorganische Bestandteile, vorzugsweise in einer Gesamtmenge von 5 bis 40 Gew.-%, bevorzugt in einer Gesamtmenge von 10 bis 30 Gew.-%, jeweils bezogen auf die Gesamtmasse der dentalen Zusammensetzung,
wobei bevorzugt der, einer, mehrere oder sämtliche der anorganischen Bestandteile mikrofeine Füllstoffe sind, vorzugsweise ausgewählt aus der Gruppe bestehend aus pyrogene Kieselsäure und Fällungskieselsäure,
wobei besonders bevorzugt das Massenverhältnis von der Gesamtmenge an photopolymerisierbaren Monomeren ausgewählt aus der Gruppe bestehend aus Acrylaten und Methacrylaten gemäß Komponente (a) zu der Gesamtmenge an mikrofeinen Füllstoffen ausgewählt aus der Gruppe bestehend aus pyrogene Kieselsäure und Fällungskieselsäure im Bereich von 3 : 1 bis 4 : 1 liegt.

15. Verwendung von *α*-Terpinen oder *γ*-Terpinen
als Molekulargewichtsregler bei der Polymerisation von Monomeren ausgewählt aus der Gruppe bestehend aus Acrylaten und Methacrylaten, vorzugsweise aus der Gruppe der Methacrylate,
zur Herstellung einer dentalen Abdeckmasse.

16. Kit umfassend:
- eine Zusammensetzung nach einem der Ansprüche 1 bis 14 und
- ein Mittel zum Behandeln eines Zahnes, welches bei Kontakt mit einem Zahn diesen und/oder bei Kontakt mit dem Zahnfleisch dieses verändert,
wobei vorzugsweise das Mittel zum Behandeln eines Zahnes ein Mittel zur dentalen Zahnbleichung ist.

17. Verwendung einer dentalen Zusammensetzung nach einem der Ansprüche 1 bis 14 zum
- Herstellen einer Schutzschicht zum Abdecken von Zahnfleisch und/oder Zähnen und zum Isolieren des Zahnfleisches bzw. der Zähne gegenüber benachbarten Zahnpartien
und/oder
- in einem Verfahren zum kosmetischen Behandeln eines oder mehrerer Zähne.

## Claims

1. A photopolymerisable dental composition as a dental covering material for application to and for the isolation and protection of gums and/or teeth during dental treatment, comprising:
(a) one or more photopolymerisable monomers selected from the group consisting of acrylates and methacrylates,
(b) one or more photoinitiators,
(c) one or more molecular weight regulators selected from the group consisting of α-terpinene and γ-terpinene
and optionally one or more further additives.

2. The dental composition according to claim 1, comprising
(a) one or more photopolymerisable monomers selected from the group of the methacrylates
and/or
(b) one or more photoinitiators selected from the group consisting of alpha-diketones, benzoin alkyl ethers, thioxanthones, benzophenones, acyl phosphine oxides, acetophenones, ketals, titanocenes, borates and sensitising dyes.

3. The dental composition according to one of the preceding claims, comprising
component (a) in a quantity of from 50 to 90 wt.%, preferably in a quantity of from 60 to 80 wt.%,
component (b) in a quantity of from 0.01 to 1 wt.%, preferably from 0.05 to 0.5 wt.%, preferably from 0.1 to 0.3 wt.%, and
component (c) in a quantity of from 0.075 to 7.5 wt.%, preferably from 0.125 to 3 wt.%, preferably from 0.2 to 1.5 wt.%,
based in each case on the total mass of the dental composition.

4. The dental composition according to one of the preceding claims, wherein component (a) comprises:
(a) (i) one or more dimethacrylate monomers
and/or
(a) (ii) one or more acid group-containing monomers,
and wherein component (a) preferably comprises both component (a) (i) and component (a) (ii),
wherein component (a) (i) preferably contains one or more dimethacrylate monomers selected from the group consisting of ethylene glycol dimethacrylate (EGDMA), 1,6-hexanediol dimethacrylate (HEDMA), triethylene glycol dimethacrylate (TEGDMA), 1,12-dodecanediol dimethacrylate (DODMA), ethoxylated bisphenol A dimethacrylate, polyethylene glycol dimethacrylate (PEGDMA), 7,7,9-trimethyl-4,13-dioxo-5,12-diazahexadecane 1,16-dioxydimethacrylate (UDMA), butanediol dimethacrylate, tetraethylene glycol dimethacrylate, neopentyl glycol dimethacrylate, 2-hydroxypropyl 1,3-dimethacrylate, 3-hydroxypropyl 1,2-dimethacrylate, pentaerythritol dimethacrylate, glycerol dimethacrylate, bisphenol A glycidyl methacrylate (Bis-GMA) and dimethacrylates of dihydroxymethyltricyclo[5.2.1.0^{2,6}]decane and/or
wherein component (a) (ii) preferably contains one or more acid group-containing monomers which have a carboxylic acid function, a phosphoric acid function, a phosphonic acid function, a sulfonic acid function and/or a thiophosphoric acid function, preferably a phosphoric acid function.

5. The dental composition according to claim 4,
**characterised in that**
component (a) (ii)
- contains one or more phosphoric acid group-containing monomers selected from the group consisting of 2-(meth)acryloyloxyethyl dihydrogenphosphate, bis[2-(meth)acryloyloxyethyl] hydrogenphosphate, 2-(meth)acryloyloxyethylphenyl hydrogenphosphate, 6-(meth)acryloyloxyhexyl dihydrogenphosphate, 10-(meth)acryloyloxydecyl dihydrogenphosphate (MDP), 6-(meth)acryloyloxyhexylphenyl hydrogenphosphate, 10-(meth)acryloyloxydecyl dihydrogenphosphate, 1,3-di(meth)acryloyloxypropane 2-dihydrogenphosphate, 1,3-di(meth)acryloyloxypropane-2-phenyl hydrogenphosphate and bis[5-(2-(meth)acryloyloxyethoxycarbonyl)heptyl] hydrogenphosphate,
and/or
- contains one or more carboxylic acid group-containing monomers selected from the group consisting of 4-(meth)acryloxyethyl trimellitic acid (4-MET), 4-(meth)acryloxyethyl trimellitic anhydride (4-META), 4-(meth)acryloxydecyl trimellitic acid, 4-(meth)acryloxydecyl trimellitic anhydride, 11-(meth)acryloyloxy-1,1-undecanedicarboxylic acid, 1,4-di(meth)acryloyloxypyromellitic acid, 2-(meth)acryloyloxyethyl maleic acid, 2-(meth)acryloyloxyethyl phthalic acid and 2-(meth)acryloyloxyethyl hexahydrophthalic acid.

6. The dental composition according to one of claims 4 to 5, comprising component (a) (i) in a quantity of from 42 to 89.5 wt.%, preferably in a quantity of from 56 to 79 wt.%,
and/or
component (a) (ii) in a quantity of from 0.5 to 8 wt.%, preferably in a quantity of from 1 to 4 wt.%,
based in each case on the total mass of the dental composition.

7. The dental composition according to one of the preceding claims, further comprising
(d) one or more polymerisation inhibitors for increasing the storage stability of the composition, preferably selected from the group consisting of hydroquinone monomethyl ether (HQME), phenols, preferably 2,6-di-tert.-butyl-4-methylphenol (BHT) and tert.-butylhydroxyanisole (BHA), 2,2-diphenyl-1-picrylhydrazyl radicals, galvinoxyl radicals, triphenylmethyl radicals, 2,3,6,6-tetramethylpiperidinyl-1-oxyl radical (TEMPO) and derivatives thereof, and phenothiazine and derivatives thereof,
preferably comprising
component (d) in a quantity of from 10 to 2000 ppm, preferably 50 to 1500 ppm, preferably 300 to 1000 ppm,
based in each case on the total mass of the dental composition, wherein preferably the ratio of the total mass of component (c) to the total mass of component (d) is greater than 1, preferably greater than 2, preferably greater than 4.

8. The dental composition according to one of the preceding claims, wherein the dental composition can be applied by means of a dental syringe
and/or
when applied in the mouth, has a rheological flow behaviour and a stability such that no running or spreading of the applied composition takes place.

9. The dental composition according to one of the preceding claims, **characterised in that** the dental composition, after application to teeth and/or gums, can be transformed by light curing into an elastic material
- which can be pulled off from said teeth or said gums while leaving behind no residue
and/or
- which has a modulus of elasticity of < 4000 MPa, preferably < 2000 MPa, preferably < 1000 MPa.

10. The dental composition according to one of the preceding claims, **characterised in that** the viscosity of the dental composition is greater than or equal to 10,000 mPas, preferably greater than or equal to 15,000 mPas and further preferably greater than or equal to 20,000 mPas, and/or the viscosity of the dental composition is in the range of from 10,000 mPas to 80,000 mPas, preferably in the range of from 20,000 mPas to 35,000 mPas.

11. The dental composition according to one of the preceding claims, **characterised in that** the dental composition cannot be transformed by light curing into an elastic material which has a modulus of elasticity of > 2000 MPa and preferably cannot be transformed by light curing into an elastic material which has a modulus of elasticity of > 1000 MPa.

12. The dental composition according to one of the preceding claims, **characterised in that** the dental composition has a depth of cure in the range of from 1.2 to 2.2 mm, preferably 1.5 to 1.8 mm.

13. The dental composition according to one of the preceding claims,
**characterised in that** the dental composition
can be transformed by light curing into an elastic material which has a bending strength of less than 50 MPa, preferably of less than 47 MPa,
and/or
cannot be transformed by light curing into an elastic material which has a bending strength of more than 50 MPa and particularly preferably cannot be transformed by light curing into an elastic material which has a bending strength of more than 47 MPa.

14. The dental composition according to one of the preceding claims, further comprising
(e) one or more inorganic components, preferably in a total quantity of from 5 to 40 wt.%, preferably in a total quantity of from 10 to 30 wt.%, based in each case on the total mass of the dental composition,
wherein preferably the inorganic component or one, a plurality or all of the inorganic components are microfine fillers, preferably selected from the group consisting of pyrogenic silica and precipitated silica,
wherein particularly preferably the mass ratio of the total quantity of photopolymerisable monomers selected from the group consisting of acrylates and methacrylates of component (a) to the total quantity of microfine fillers selected from the group consisting of pyrogenic silica and precipitated silica is in the range of from 3:1 to 4:1.

15. Use of α-terpinene or γ-terpinene
as a molecular weight regulator in the polymerisation of monomers selected from the group consisting of acrylates and methacrylates, preferably from the group of the methacrylates,
for producing a dental covering material.

16. A kit comprising:
- a composition according to one of claims 1 to 14 and
- an agent for treating a tooth, which changes a tooth upon contact therewith and/or changes the gum upon contact therewith,
wherein the agent for treating a tooth is preferably a dental tooth whitening agent.

17. Use of a dental composition according to one of claims 1 to 14 for
- producing a protective layer for covering gums and/or teeth and for isolating the gums or teeth, as the case may be, from adjacent tooth parts
and/or
- in a method for the cosmetic treatment of one or more teeth.

## Revendications

1. Composition dentaire photopolymérisable comme masse de recouvrement dentaire pour l'application sur, et l'isolement et la protection de la gencive et/ou des dents pendant un traitement dentaire, comprenant :
(a) un ou plusieurs monomères photopolymérisables choisis dans le groupe consistant en acrylates et méthacrylates,
(b) un ou plusieurs photo-initiateurs,
(c) un ou plusieurs régulateurs du poids moléculaire choisis dans le groupe consistant en α-terpines et γ-terpines
et éventuellement un ou plusieurs autres additifs.

2. Composé dentaire selon la revendication 1, comprenant
(a) un ou plusieurs monomères photopolymérisables choisis dans le groupe des méthacrylates,
et/ou
(b) un ou plusieurs photo-initiateurs choisis dans le groupe consistant en alpha-dicétones, benzoïne-alkyléthers, thioxanthones, benzophénones, oxyde d'acylphosphine, acétophénones, cétals, titanocènes, borates et colorants sensibilisants.

3. Composition dentaire selon l'une des revendications précédentes, comprenant
des composants (a) en une quantité de 50 à 90 % en poids, de préférence en une quantité de 60 à 80 % en poids,
des composants (b) en une quantité de 0,01 à 1 % en poids, de préférence de 0,05 à 0,5 % en poids, de manière préférée de 0,1 à 0,3 % en poids, et
des composants (c) en une quantité de 0,075 à 7,5 % en poids, de préférence de 0,125 à 3 % en poids, de manière préférée de 0,2 à 1,5 % en poids,
respectivement par rapport à la masse totale de la composition dentaire.

4. Composition dentaire selon l'une des revendications précédentes, dans laquelle le composant (a) comprend :
(a) (i) un ou plusieurs monomères diméthacrylate et/ou
(a) (ii) un ou plusieurs monomères contenant des groupes acides,
et dans laquelle le composant (a) comprend de préférence aussi bien un composant (a) (i) que (a) (ii),
dans laquelle de préférence le composant (a) (i) contient un ou plusieurs monomères diméthacrylates choisis dans le groupe consistant en éthylèneglycol diméthacrylate (EGDMA), 1,6-hexanediol-diméthacrylate (HEDMA), triéthylèneglycol-diméthacrylate (TEGDMA), 1,12-dodécanediol-diméthacrylate (DODMA), bisphénol-A-diméthacrylate éthoxylé, polyéthylèneglycol-diméthacrylate (PEGDMA), 7,7,9-triméthyl-4,13-dioxo-5,12-diazahexadécane-1,16-dioxydiméthacrylate (UDMA), butanediol-diméthacrylate, tétraéthylèneglycol-diméthacrylate, néopentylglycol-diméthacrylate, 2-hydroxypropyl-1,3-diméthacrylate, 3-hydroxypropyl-1,2-diméthacrylate, pentaérythritol-diméthacrylate, glycérine-diméthacrylate, bisphénol-A-glycidyl-méthacrylate (bis-GMA) et diméthacrylates de dihydroxyméthyltricyclo[5.2.1.0^{2,6}]décane
et/ou
dans laquelle de préférence le composant (a) (ii) contient un ou plusieurs monomères contenant un groupe acide qui présentent une fonction d'acide carboxylique, une fonction d'acide phosphorique, une fonction d'acide phosphonique, une fonction d'acide sulfonique et/ou une fonction d'acide thiophosphorique, de préférence une fonction d'acide phosphorique.

5. Composition dentaire selon la revendication 4, **caractérisée en ce que**
le composant (a) (ii)
- contient un ou plusieurs monomères contenant un groupe d'acide phosphorique qui sont choisis dans le groupe consistant en 2-(méth)acryloyloxyéthyl-dihydrogénophosphate, bis[2-(méth)acryloyloxyéthyl]-hydrogénophosphate, 2-(méth)acryloyloxyéthylphényl-hydrogénophosphate, 6-(méth)-acryloyloxyhexyl-dihydrogénophosphate, 10-(méth)acryloyloxydécyl-dihydrogénophosphate (MDP), 6-(méth)-acryloyloxy-hexylphényl-hydrogénophosphate, 10-(méth)acryloyloxy-décyl-dihydrogénophosphate, 1,3-di(méth)acryloyloxy-propan-2-dihydrogénophosphate, 1,3-di-(méth)acryloyl-oxypropane-2-phénylhydrogénophosphate et bis[5-(2-(méth)acryloyloxyéthoxycarbonyl)heptyl]hydrogénophospha te,
et/ou
- un ou plusieurs monomères contenant un groupe d'acide carboxylique qui sont choisis dans le groupe consistant en acide 4-(méth)acryloxyéthyltrimellithique (4-MET), anhydride d'acide 4-(méth)acryloxyéthyltrimellitique (4-META), acide 4-(méth)acryloxydécyltrimellitique, anhydride d'acide 4-(méth)acryloxydécyltrimellitique, acide 11-(méth)acryloyloxy-1,1-undécanedicarboxylique, acide 1,4-di(méth)acryloyloxypyromellithique, acide 2-(méth)acryloyloxyéthylmaléique, acide 2-(méth)acryloyloxyéthylphtalique et acide 2-(méth)acryloyloxyéthylhexadyhydrophtalique.

6. Composition dentaire selon l'une des revendications 4 à 5, comprenant
le composant (a) (i) en une quantité de 42 à 89,5 % en poids, de préférence en une quantité de 56 à 79 % en poids,
et/ou
le composant (a) (ii) en une quantité de 0,5 à 8 % en poids, de préférence en une quantité de 1 à 4 % en poids,
respectivement par rapport à la masse totale de la composition dentaire.

7. Composition dentaire selon l'une des revendications précédentes, comprenant en outre
(d) un ou plusieurs inhibiteurs de polymérisation pour augmenter la stabilité au stockage de la composition, choisis de préférence dans le groupe consistant en monométhyléther d'hydroquinone (HQME), phénols, parmi lesquels de préférence le 2,6-di-tert-butyl-4-méthylphénol (BHT) et le tert-butylhydroxyanisol (BHA), des radicaux 2,2-diphényl-1-picrylhydrazyle, des radicaux galvinoxyle, des radicaux triphénylméthyle, le radical 2,3,6,6-tétraméthylpipéridinyl-1-oxyle (TEMPO) et leurs dérivés et la phénothiazine et ses dérivés,
comprenant de préférence
le composant (d) en une quantité de 10 à 2000 ppm, de préférence de 50 à 1500 ppm, de manière préférée de 300 à 1000 ppm,
respectivement par rapport à la masse totale de la composition dentaire,
dans laquelle, de manière préférée, le rapport de la masse totale du composant (c) à la masse totale du composant (d) est supérieur à 1, de préférence supérieur à 2, de préférence supérieur à 4.

8. Composition dentaire selon l'une des revendications précédentes, dans laquelle la composition dentaire peut être appliquée à l'aide d'une seringue dentaire
et/ou
présente, lors d'une application dans la bouche, un comportement rhéologique de viscoélasticité et une stabilité telles qu'il ne se produit pas de coulage ou d'étalement de la composition appliquée.

9. Composition dentaire selon l'une des revendications précédentes, **caractérisée en ce que** la composition dentaire, après application sur des dents et/ou gencives, peut être convertie par photodurcissage en un matériau élastique
- qui peut être retiré sans résidus desdites dents ou de ladite gencive
et/ou
- possède un module d'élasticité < 4000 MPa, de préférence < 2000 MPa, de manière préférée < 1000 MPa.

10. Composition dentaire selon l'une des revendications précédentes, **caractérisée en ce que** la viscosité de la composition dentaire est supérieure ou égale à 10 000 mPa, de préférence supérieure ou égale à 15 000 mPa et de manière davantage préférée supérieure ou égale à 20 000 mPa et/ou la viscosité de la composition dentaire se situe dans la plage de 10 000 mPa à 80 000 mPa, de manière préférée dans la plage de 20 000 mPa à 35 000 mPa.

11. Composition dentaire selon l'une des revendications précédentes, **caractérisée en ce que** la composition dentaire ne peut pas être convertie par photodurcissage en un matériau élastique qui possède un module d'élasticité > 2000 MPa, de préférence ne peut pas être convertie en un matériau élastique qui possède un module d'élasticité > 1000 MPa.

12. Composition dentaire selon l'une des revendications précédentes, **caractérisée en ce que** la composition dentaire possède une profondeur de durcissement dans la plage de 1,2 à 2,2 mm, de préférence de 1,5 à 1,8 mm.

13. Composition dentaire selon l'une des revendications précédentes, **caractérisée en ce que** la composition dentaire
peut être convertie par photodurcissage en un matériau élastique qui possède une résistance à la flexion inférieure à 50 MPa, de préférence inférieure à 47 MPa,
et/ou
ne peut pas être convertie par photodurcissage en un matériau élastique qui possède une résistance à la flexion supérieure à 50 MPa, de manière particulièrement préférée ne peut pas être convertie par photodurcissage en un matériau élastique qui possède une résistance à la flexion supérieure à 47 MPa.

14. Composition dentaire selon l'une des revendications précédentes, comprenant en outre
(e) un ou plusieurs composants inorganiques, de préférence en une quantité totale de 5 à 40 % en poids, de manière préférée en une quantité totale de 10 à 30 % en poids, respectivement par rapport à la masse totale de la composition dentaire,
dans laquelle de manière préférée le, un, plusieurs ou la totalité des composants inorganiques sont des agents de charge microfins, choisis de préférence dans le groupe consistant en acide silicique pyrogène et acide silicique précipité,
dans laquelle de manière particulièrement préférée, le rapport en masse de la quantité totale de monomères photopolymérisables choisis dans le groupe consistant en acrylates et méthacrylates selon le composant (a), à la quantité totale d'agents de charge microfins choisis dans le groupe consistant en acide silicique pyrogène et acide silicique précipité, se situe dans la plage de 3:1 à 4:1.

15. Utilisation d'α-terpines ou de γ-terpines
comme régulateur du poids moléculaire dans la polymérisation de monomères choisis dans le groupe consistant en acrylates et méthacrylates, de préférence dans le groupe des méthacrylates,
pour la production d'une masse de recouvrement dentaire.

16. Kit comprenant :
- une composition selon l'une des revendications 1 à 14 et
- un moyen de traitement d'une dent, qui, par contact avec une dent, modifie celle-ci et/ou, par contact avec la gencive, modifie celle-ci,
dans lequel, de préférence, le moyen de traitement d'une dent est un moyen de blanchiment dentaire de la dent.

17. Utilisation d'une composition dentaire selon l'une des revendications 1 à 14, pour
- la production d'une couche protectrice pour le recouvrement de la gencive et/ou des dents et pour l'isolement de la gencive et/ou des dents par rapport à des parties dentaires voisines
et/ou
- dans un procédé de traitement esthétique d'une ou plusieurs dents.
